# EUROPEAN PATENT SPECIFICATION

(11) **EP 1 704 252 B1**
(45) Date of publication and mention of the grant of the patent: **14.05.2008**
(21) Application number: 05701224.7
(22) Date of filing: 14.01.2005
(51) Int. Cl.: C12Q 1/68

(54) **POLYMORPHISMS OF CHROMOSOME 9 IMPLICATED IN PREMATURE CANITIES**
MIT VORZEITIGER CANITIES IN VERBINDUNG GEBRACHTE POLYMORPHISMEN VON CHROMOSOM 9
POLYMORPHISMES DU CHROMOSOME 9 IMPLIQUES DANS LES CANITIES PRECOCES

(30) Priority: 15.01.2004 FR 0400371; 12.02.2004 US 543544 P
(43) Date of publication of application: 27.09.2006
(73) Proprietor: L'ORÉAL, 75008 Paris (FR)
(72) Inventor: DE LACHARRIERE, Olivier, F-75015 Paris (FR); BLOUIN, Jean-Louis, F-74100 Ville-La-Grand (FR); DELOCHE, Claire, F-75015 Paris (FR); ANTONARAKIS, Stylianos, CH-1205 Geneva (CH); DERMITZAKIS, Emmanouil, CH-1207 Geneve (CH)
(74) Representative: Sellin, Carole
(86) International application number: PCT/EP2005/000819
(87) International publication number: WO 2005/068652

(56) References cited:
- WO-A-96/36691
- WO-A-02/097047
- WO-A-20/04007742
- FR-A- 2 842 104
- DATABASE HGVBASE EMBL-EBI; 7 August 2003 (2003-08-07), "Human Genic Bi-Allelic Sequences Database" XP002290894
- DATABASE HGVBASE EMBL-EBI; 7 August 2003 (2003-08-07), XP002292757 Database accession no. RS306534
- DATABASE HGVBASE EMBL-EBI; 7 August 2003 (2003-08-07), XP002292758 Database accession no. RS3739902
- DATABASE HGVBASE EMBL-EBI; 7 August 2003 (2003-08-07), XP002292759 Database accession no. RS575916
- DATABASE HGVBASE EMBL-EBI; 7 August 2003 (2003-08-07), XP002292760 Database accession no. RS365297
- DATABASE HGVBASE EMBL-EBI; 7 August 2003 (2003-08-07), XP002292761 Database accession no. RS2583805
- DATABASE HGVBASE EMBL-EBI; 7 August 2003 (2003-08-07), XP002292762 Database accession no. RS377090
- OETTING W S ET AL: "Molecular basis of albinism: mutations and polymorphisms of pigmentation genes associated with albinism." HUMAN MUTATION. UNITED STATES 1999, vol. 13, no. 2, 1999, pages 99-115, XP008018509 ISSN: 1059-7794
- BARSH G S: "The genetics of pigmentation: from fancy genes to complex traits" TRENDS IN GENETICS, ELSEVIER SCIENCE PUBLISHERS B.V. AMSTERDAM, NL, vol. 12, no. 8, 1 August 1996 (1996-08-01), pages 299-305, XP004037138 ISSN: 0168-9525
- HEMMINKI A ET AL: "Localization of a susceptibility locus for Peutz-Jeghers syndrome to 19p using comparative genomic hybridization and targeted linkage analysis" NATURE GENETICS, NEW YORK, NY, US, vol. 15, no. 1, 15 January 1997 (1997-01-15), pages 87-90, XP001105579 ISSN: 1061-4036
- TASSABEHJI M ET AL: "Waardenburg syndrome type 2 caused by mutations in the human microphthalmia (MITF) gene" NATURE GENETICS, NEW YORK, NY, US, vol. 8, no. 3, November 1994 (1994-11), pages 251-255, XP000909097 ISSN: 1061-4036

### SEQUENCE LISTING

<110> L'OREAL
<120> Polymorphisms of chromosome 9 implicated in premature canities
<130> B5892A - CA/CS
<150> FR 0400371
   <151> 2004-01-15
<150> US 60/543,544
   <151> 2004-02-12
<160> 6
<170> Patent In version 3.1
<210> 1
   <211> 41
   <212> DNA
   <213> Homo sapiens
<400> 1
   gggaaggcac gcagaccagg ygggatgatc attacagtga a 41
<210> 2
   <211> 41
   <212> DNA
   <213> Homo sapiens
<400> 2
   tttttaagta ttgaagactt wcctcgacta acgtgattta g 41
<210> 3
   <211> 41
   <212> DNA
   <213> Homo sapiens
<400> 3
   aagacccacg ataggagcaa saagctggga gtgagagagg t 41
<210> 4
   <211> 41
   <212> DNA
   <213> Homo sapiens
<400> 4
   tataataagg gggtaaatta kgaaacattc atacactaga a 41
<210> 5
   <211> 41
   <212> DNA
   <213> Homo sapiens
<400> 5
   ccgagtctct gcctgattgc sgattacagg ctacacagca c 41
<210> 6
   <211> 41
   <212> DNA
   <213> Homo sapiens
<400> 6
   acaggaaatc cttatatagc yctcttatag ttgacaaatg g 41

## Description

Do what one may to abolish or reduce them, the effects of ageing are a preoccupation which does not cease to grow. In this context, causing white hair judged to be unsightly to disappear by treatment with colour shampoos has become and will continue to be a very widespread activity. It is clear, however, that even though such treatment actually makes it possible to abolish the appearance of the phenomenon, it has no effect whatever on the causes. As a result, this solution is temporary and must be frequently renewed.

In this context, the inventors have chosen to explore the appearance of white hair, or canities, from a completely new angle, that of genetics.

In fact, exploring canities from the point of view of its genetics makes it possible to identify the underlying mechanisms of depigmentation. That also makes it possible to identify the genes that are implicated in canities. This identification opens the door to very many applications in the field of hair care, whether cosmetic, therapeutic or diagnostic.

It is highly innovative to try to identify the regions of the genome responsible for canities by genetic linkage analysis whereas other studies are more concerned with deciphering the biochemistry of canities.

The inventors have chosen to take advantage of the hypothesis suggested a long time ago concerning the hereditary character of premature canities (PC), or the appearance of white hair early in life. The familial character of premature whitening of the hair in certain people is in fact readily observable.

A considerable obstacle to the implementation of reverse genetics relates to the precise definition of the phenotype. A complete definition of the phenotype under study is in fact necessary. In order to guarantee the best chances of success for the identification of the genes in this case, the choice and composition of the sample used in the present invention are the result of the application of a rigorous protocol for the assignment of the phenotype and the selection of the families. The "premature canities" phenotype was assigned only to individuals who had white hair before they were 25 years old and half of whose scalp hair was grey at 30 years of age.

In addition, it is probable that, on the one hand, premature canities has a multigenic, and not a monogenic, origin and, on the other hand, that environmental factors have an influence on the phenotype. In fact the subject requires the definition of a set of causes that predispose to premature canities. In this context, reverse genetics is not usually a procedure recommended by geneticists. It is therefore original on the part of the inventors to have used this method.

The results of this work have enabled the inventors, in a first stage, to define chromosomal and/or genomic regions comprising genes implicated with high probability in canities. In the present application, the inventors have demonstrated polymorphisms within the genes DDX31 and GTF3C4 of chromosome 9, statistically implicated in canities.

The subject of the present invention is based, on the one hand, on the identification of 4 SNP (single nucleotide polymorphism) polymorphisms designated rs306534, rs3739902, rs575916 and rs365297 implicated in the predisposition to premature canities and, on the other, on the identification of a combination of the polymorphisms rs3739902, rs2583805 and rs377090 defining a haplotype implicated in the predisposition to premature canities. The present invention also relates to the use of these markers in processes and kits in the fields of cosmetics, therapeutics and diagnosis.

In the case of the fields of therapy and cosmetics, the present invention successively relates to the use of at least one of the 4 SNP markers rs306534, rs3739902, rs575916 and rs365297 for carrying out a diagnosis, a process for diagnosing a predisposition to premature canities, the use of a means for determining the alleles of the 4 markers in order to make a diagnosis and a kit for the diagnosis.

The present invention also relates to a process for the diagnosis of the predisposition to premature canities based on the haplotype defined by the markers rs3739902, rs2583805 and rs377090.

Finally the diagnosis of a predisposition to premature canities in a non-human mammal, based on the use of the information contained in the genomic region of the said mammal homologous to the region of the human chromosome 9 included between the markers rs306534 and rs365297, is disclosed.

### GLOSSARY

In the context of the present invention the terms used have the following meaning:
By polynucleotide fragment is meant any molecule resulting from the linear linking of at least two nucleotides, this molecule being possibly single-stranded, double-stranded or triple-stranded. It may therefore be a double-stranded DNA molecule, a single-stranded DNA molecule, an RNA, a duplex of single-stranded DNA-RNA, a DNA-RNA triplex or any other combination. The polynucleotide fragment may be naturally occurring, recombinant or also synthetic. When the polynucleotide fragment comprises complementary strands, the complementarity is not necessarily perfect, but the affinity between the different strands is sufficient to allow the establishment of stable links of the Watson-Crick type between the two strands.

Although the matching of the bases is preferably of the Watson-Crick type, other types are not excluded, such as a matching of the Hoogsteen type or reverse Hoogsteen type.

It is considered that the sequence S of a molecule "corresponds" to the sequence of a given DNA molecule D if it is possible to deduce the sequence of the bases of S from that of the given DNA molecule D by one of the following processes
1. by identity, or
2. by identity but by changing all or some of the thymines to uracils, or
3. by complementarity, or
4. by complementarity but by changing all or some of the thymines to uracils.

In addition, it is considered that two sequences remain "corresponding" if overall less than one error in ten is introduced in one of the preceding processes (complementarity or identity, with or without T,U exchange), and preferably less than one error in 100. Consequently, the two molecules also necessarily have similar lengths, since the maximum variation in length is 10% of the accepted level of error; they preferably have a difference in length of less than 1%.

This definition does not assume that the two molecules are of the same kind, in particular as regards their skeleton, there is uniquely a correspondence between their sequences.

For example, two identical DNA sequences "correspond" to each other.

Similarly, if these two sequences are substantially identical, i.e. identical to more than 90%, they correspond to each other. An RNA sequence, derived from the transcription of any DNA molecule, "corresponds" to the sequence of this DNA molecule. Similarly, a synthetic sequence, for example a DNA-RNA hybrid, may correspond to a DNA sequence. The same holds true between a DNA sequence and the anti-sense RNA that targets this sequence.

In the same schema, it is considered that the sequence S of a DNA molecule **"corresponds"** to the sequence of a given DNA molecule D if it is possible to deduce the sequence S from that of the given DNA molecule by the process 1 or 3 uniquely. The same latitude is allowed concerning the possibility of introducing errors in to these processes, i.e. it is considered that two DNA sequences remain "corresponding" if overall less than one error in 10 is introduced in the processes of complementarity or of identity, and preferably less than one error in 100.

A **genetic marker** is a detectable DNA sequence. In human genetics, markers are specific sequences of the DNA that are capable of assuming different forms depending on the individuals. This polymorphism of the markers makes it possible to follow their transmission in the context of genealogical trees.

Among the conventional **markers,** it is possible to identify two large classes of markers which are the microsatellite markers and the SNPs (Single Nucleotide Polymorphisms).

A **microsatellite** is a repeated DNA sequence, constituted of a relatively simple motif:
most frequently a di-, tri- or tetranucleotide. The number of repeats of the same motif changes depending on the individuals and may vary from several units (a dozen at least for a dinucleotide) up to more than one hundred. These sequences are scattered more or less everywhere throughout the genome in an almost random manner but at sites identical from one individual to another. They are very abundant (about one every 10,000 nucleotides = 10 kb) and they are very polymorphic. It is the variation in length of the tandem repeat (number of repeats) which constitutes the marker. These microsatellite sequences are hence very much used as genetic markers.

Usually, there is no explicit link between a microsatellite marker and a gene, except a co-localisation. According to present knowledge and apart from a few rare cases of intragenic markers associated with certain diseases, the length of a tandem repeat is unrelated to the role of a gene. In the context of the present invention, the microsatellite markers are tools for localising the genes implicated in premature canities. As there is much less polymorphism in the genes than in the markers, a genic allele will be represented by several alleles of the same microsatellite marker.

There are different methods for defining the localisation of specific DNA sequences along the chromosomes. The physical unit of measure is the number of base pairs. However, the **centimorgan** is often used, that is a unit of recombination, thus a genetic unit of measure and not a physical one. Two specific sequences on the same chromosome are separated by a centimorgan if there is one chance in a hundred that they recombine during meiosis. A centimorgan is approximately equivalent to 10⁶ base pairs.

Another method for localising specific DNA sequences along the chromosomes consists of defining their position relative to markers distributed along the chromosomes and the position of which is completely defined and known. Very much used markers are microsatellite markers for which very complete mappings exist. In particular the GDB "Genome Database" is a data bank, known world-wide to index among other things the STSs (Sequence tagged sites), specific and unique landmarks on the DNA which include the microsatellites. The DxSxxxx codes (for example D6S257), serving to identify these markers, are their access numbers in the GDB. These codes are an unambiguous and universal means of identification because only the GDB assigns this type of code. As such microsatellite markers can be found about every 10 kb, it is thus possible to define the position of every sequence to about 10 kb, by indicating the microsatellite markers framing it.

A SNP (Single Nucleotide Polymorphism) is a polymorphism which affects a single base of the DNA. It is the most widespread form of polymorphism in the human genome, and it is also characterised by high stability on transmission. Most of these polymorphisms have no functional implications. On average 1 SNP is found for every 100 base pairs. Knowledge of these SNPs makes it possible to construct a map of the human genome and the SNPs then serve as true markers of the genome, all the more so because they mutate slowly and have little chance of reappearing in a recurrent manner.

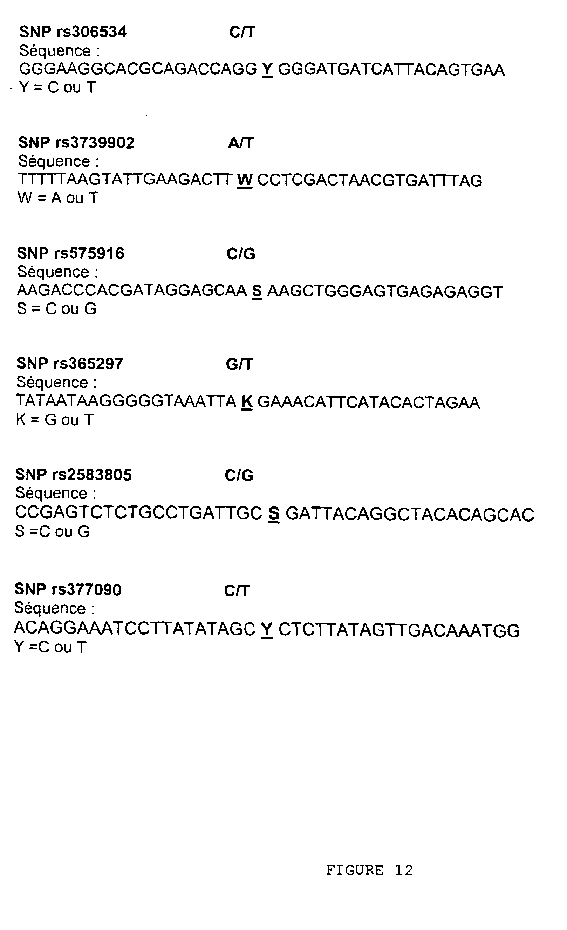

The SNPs are catalogued and referenced in different, freely accessible banks, in particular in the GDB. The human genomic sequences flanking the SNPs rs306534, rs3739902, rs575916, rs365297, rs2583805 and rs377090, making it possible to localise them with certainty, are illustrated in Figure 12.

By chromosomal region **between two markers** (or included between two markers, or comprised between two markers) is meant the entire sequence included between these two markers, the termini, thus the sequence of the markers, being included.

In reverse **genetics,** the indices making it possible to localise a gene originate from the comparison of the transmission of a phenotype, supposedly induced by a mutated gene or by a given allele, with the transmission of known markers within the same family. These co-segregation data of a phenotype and a marker make it possible to establish a genetic linkage analysis.

The co-transmission of a phenotype and a marker suggest that the genes responsible for the phenotype and the marker are physically close to each other on the chromosome. The linkage is defined by the analysis of the transmission schema of a gene and a marker in families that lend themselves to it.

The **linkage analysis** is based on the co-transmission of certain forms of markers with the defective or modified form of a gene. But it is an indirect analysis in the sense that, on the one hand, during a first step, a phenotype is associated with the defective or modified form of a gene. An error in the assignment of certain phenotypes falsifies the study. On the other hand, this study is based on statistics, these statistics being based on the analysis of a sample of the population, it is thus a survey. Finally, it should be noted that when it is possible to associate a particular allele of the marker with an allele of the gene (in fact a phenotype), this association is a priori only valid for inter-familial samples.

The result of the linkage analyses obviously depends on the degree of linkage between the marker and the locus of the disease. Five centimorgans (5 cM) is considered as a linkage minimum for a diagnosis. A linkage of 5 cM signifies that there are 95% chances to arrive at a correct conclusion and only one chance in 20 that a recombination has occurred between the marker and the locus of the disease.

By the term **gene,** in the context of the present invention, is meant not only the strictly coding part but also the non-coding parts such as the introns and the regulatory parts at 5' and 3', the UTRs (UnTranslated Region), in particular the promoter(s), enhancer(s) etc... associated.

A **haplotype** is a combination of given alleles present in the genetic material of an organism. Certain combinations of alleles are present at a higher frequency than the frequency obtained theoretically by random combination. This haplotype is then considered as being in **linkage disequilibrium** (LD).

It is considered that a polymorphism is **statistically implicated** in the appearance of a phenotype when the frequency of this polymorphism in persons having the phenotype is higher than the frequency calculated if these two events were independent.

WO02/097047 discloses a method for inferring a genetic pigmentation trait of a human subject based, in part, on the identification of single nucleotide polymorphisms that, alone or in combination, allow an inference to be drawn as to a genetic pigmentation trait such as hair shade, hair color, eye shade, or eye color.

The inventors have identified a chromosomal region belonging to chromosome 9 and which is implicated in premature canities. The inventors have more particularly demonstrated the implication of certain polymorphisms belonging to this chromosomal region, called polymorphisms of the invention.

According to a **first** aspect, the invention relates to the SNP markers rs306534, rs3739902, rs575916 and rs365297 of the human chromosome 9 identified by the inventors as each being implicated in premature canities. These markers belong to the chromosomal region delimited on chromosome 9 by the microsatellite marker D9S290 and the telomeric region (telomer of the long arm) and are located within the genes DDX31 and GTF3C4.

The invention covers the use of at least one SNP marker of the human chromosome 9 for the diagnosis of a predisposition to premature canities in an individual where the marker is chosen from the SNP markers rs306534, rs3739902, rs575916 and rs365297. The different alleles of these SNPs are illustrated in figure 12.

In the context of the present invention, it is considered that an individual is affected by premature canities when he has white hair, visible to his family circle, before the age of 25 years and that 50% of his scalp hair is grey before the age of 30 years.

Since it is very probable that environmental factors play a role in the "canities" phenotype as in that of "premature canities", the subject of the invention is to evaluate the risks of developing such a phenotype, i.e. a predisposition to premature canities.

By predisposition to premature canities is meant a probability of being affected by premature canities higher than the percentage of the population affected by premature canities. It is possible to speak of predisposition when the probability of having the premature canities trait is equal to at least 3 times the mean probability (about 1% for the white population of Western Europe).

According to a preferred embodiment of the invention, a single marker of the 4 mentioned is used for diagnostic purposes.

According to another embodiment of the invention, at least two, three or four of the SNP markers rs306534, rs3739902, rs575916 and rs365297 are used to establish the diagnosis. Since premature canities is visibly a multifactorial ailment, it is in fact sometimes very informative to combine the information obtained from different markers. Preferably, the marker rs3739902 appears in every combination of at least two markers.

Preferably, the individual is a person under 20 years of age or an individual not presenting any physical sign of premature canities.

This use according to the invention may consist in particular of determining the allele(s) of the SNP marker(s) present in the genetic material of the individual to be diagnosed. Every extract of the human body containing the DNA of the individual to be diagnosed is suitable as genetic material. It may be in particular a blood sample or skin cells or hair.

The sample containing the genetic material may be a single drop of blood which is sufficient for the implementation of a diagnosis process according to the invention. Samples of other body fluids may be used in the context of the invention. The use of some cells derived from the individual can also be envisaged.

The current procedures, well-known to the molecular biologist, may be used to carry out the determination of the alleles of the marker(s) selected; hybridisation tests are in particular very common in this type of step. Tests based on the amplification by PCR are also very widespread and can be performed on plates containing 96 or 384 samples.

Preferably, the presence of the T allele of the SNP rs306534 makes it possible to infer a predisposition of the individual to premature canities. If, on the other hand, the use relates to the SNP rs3739902, it is the presence of the T allele which makes it possible to infer a predisposition to premature canities. In the case of the SNP rs575916, it is the presence of the G allele which allows the inference of a predisposition to premature canities to be drawn. Finally, in the case of the use of the marker SNP rs365297, it is the presence of the T allele which allows the inference of a predisposition to premature canities in the individual to be drawn.

The present invention also covers a process for the diagnosis of a predisposition to premature canities in an individual. This diagnostic process comprises the determination of the alleles of a SNP marker in a sample of the genetic material of the said individual. According to this aspect of the invention, the SNP marker is selected from the SNP markers rs306534, rs3739902, rs575916 and rs365297 of the human chromosome 9.

In fact, the inventors have demonstrated the statistical linkage existing between an allele of these markers and the premature canities trait.

As the "premature canities" phenotype is transmitted to the next generation, it may prove to be important for the individuals, one of whose parents or close relative is affected, to determine whether they will or will not be similarly affected before the appearance of the symptoms. The diagnostic process according to the invention is perfectly suitable for individuals under 18 years of age.

The term "sample of genetic material" has been explained above. The specialist skilled in the art will be able to determine which sample it will be possible to use in the context of this diagnosis test, while minimising the discomfort to the individual undergoing it. If necessary, it will be possible to couple this diagnostic test with other genetic tests.

According to the process of the invention, it is possible to determine only the allele of a single SNP for the purpose of establishing the diagnosis. However, according to a preferred embodiment of the present invention, the process comprises the determination of the alleles of at least two SNPs out of the four mentioned in order to establish a diagnosis. Preferably, at least one of these SNPs is the SNP rs3739902.

In order to diagnose a predisposition to premature canities in an individual or to confirm the diagnosis, it may prove to be advantageous to compare the allelic form determined in said individual with the allelic form of the same marker(s) in other individual(s), thus serving as control(s). These individuals may be obviously affected by premature canities or, conversely, be obviously not affected by premature canities. In particular, they may be individuals more than 30 years old and having no conspicuous white hair.

It is also advantageous to select individuals for controls who are from the same geographical region as the individual to be diagnosed or who have a blood relationship with this individual, for example one of his/her parents or one of his/her siblings.

If the allele of the marker rs306534 is determined in the context of this process, then it is preferably inferred a predisposition when the allele of this marker is T.

If it is the allele of the marker rs3739902 which is determined, then the T allele makes it possible to infer a predisposition to premature canities.

In the case of the marker rs575916, it is the G allele of this marker which indicates a predisposition to premature canities.

Finally, if in the context of this process of the invention, it is the allele of the SNP rs365297 which is determined, then the inference will be drawn of a predisposition to premature canities in the presence of the T allele.

The present invention also relates to the use of a means for detecting the alleles of a SNP marker for the diagnosis of a predisposition to premature canities, wherein said means is a nucleic acid probe. According to this use of the invention, the means makes it possible to detect at least one allele of the SNP in a sample of the genetic material of the individual who must be diagnosed. The SNP of which it is desired to detect the alleles is selected from the following four SNP markers: SNP rs306534, rs3739902, rs575916 and rs365297 of the human chromosome 9.

According to a variant of this use, at least two means are used to detect the alleles of one of the four SNPs. According to another variant, several means are used making it possible to detect the alleles of at least two distinct SNPs of the SNPs rs306534, rs3739902, rs575916 and rs365297, preferably means for detecting the alleles of 3 of the 4 SNPS or means for detecting the alleles of the 4 SNPs.

It is also possible to envisage using means making it possible to detect the 2 alleles of a SNP selected from the SNPs rs306534, rs3739902, rs575916 and rs365297. Finally, it may be advantageous to use a combination of the different means described in the preceding paragraphs.

Preferably, at least one of the means makes it possible to determine an allele of the SNP rs3739902.

For detecting the allele of a SNP marker, sequencing devices which make it possible to determine the sequence of a sample of DNA or RNA may be used. In order to detect the alleles of a SNP, it is also possible to consider using nucleic acid probes which hybridise with only one of the alleles and not with the other under stringent conditions. The 4 above-mentioned SNP markers are indeed biallelic.

Highly stringent conditions making possible the hybridisation of the probe with the sample only in the case of strict complementarity can be determined by the specialist skilled in the art. They depend in particular on the length of the probe. The stringency will increase when the concentrations of salts (NaCl for example), detergents (SDS, for example), nonspecific material (salmon sperm, for example) and the temperature increase.

Such probes are, for example, polynucleotide fragments corresponding to the region surrounding (and / or comprising) the SNP marker on the human chromosome 9. Such a fragment usually has a length comprised between 10 and 50 nucleotides, preferably 12 to 35 nucleotide or 15 to 25 nucleotides. It may be a fragment of naturally occurring or synthetic DNA or RNA.

Other means or methods making it possible to detect DNA polymorphisms are well-known (allelotyping or genotyping) and sometimes make use of chip microarrays on which oligonucleotides are immobilised.

It is also possible to detect a DNA polymorphism by the PCR (Polymerase Chain Reaction) amplification procedure. In this situation, a technique is used for example which was developed from the MALDI-TOF mass spectrometry technology in which is included a step on a microarray chip which enables several tens of samples (384) to be treated at once. According to the first step of this process the samples are amplified by the PCR, the target being the DNA fragment which contains the SNP to be analysed. Then an elongation reaction (starting from a primer close to the SNP) is carried out. The length of the elongation will depend on the allele present (because elongation will be blocked by a dideoxynucleotide marker ddNTP in the case of one of the alleles which will recognise this allele by default). It is the difference in size (tiny, usually a difference of between 1 and 4 nucleotides) between the product obtained by elongation for the allele by default (A for example) and that of the other allele (G for example) detected by MALDI-TOF, which is recorded and makes it possible to type the genotype AA or AG or GG, for example. The treatment of the results obtained can be performed by means of the method "MassARRAY".

Other conventional genotyping procedures are indicated in the following references: Tang K, et al. (1999) "Chip-based genotyping by mass spectrometry", Proc. Natl. Acad. Sci. USA 96: 10016-10020; Bansal et al. (2002) "Association testing by DNA pooling - An effective initial screen", Proc. Natl. Acad. Sci. USA, Dec. 24; 99 (26): 16871-16784; Werner, M. et al. "Large scale determination of SNP allele frequencies in DNA pools using MALDI-TOF mass spectrometry", Hum. Mutat. 2002 Jul.; 20 (1): 57-64; Stoerker J, Mayo et al. "Rapid genotyping by MALDI-monitored nuclease selection from probe libraries", Nat. Biotechnol. 2000 Nov.; 18 (11): 1213-1216.

Other methods are well-known to the specialist skilled in the art, in particular that based on a mini-sequencing of the DNA in the vicinity of the polymorphic site, as a result of an elongation behind the primers in the neighbourhood of the polymorphism. It is also possible to envisage obtaining information concerning the alleles of a SNP present in a sample by PCR in real time.

Depending on the number of samples to be treated and the acceptable cost of the determination of the alleles, the specialist skilled in the art will know which technique to adopt out of the many techniques suggested or available.

When a nucleic acid probe is used, it is advantageously linked to a detection agent for example a radioactive, enzymatic, luminescent or fluorescent marker.

According to one embodiment, the means for detecting the alleles of a SNP marker makes it possible to determine the allele of rs306534. Preferably, it enables the T allele of this SNP to be detected; alternatively it enables the C allele of the marker to be detected.

According to another embodiment, the means for detecting the alleles of a SNP marker allows the allele of the marker rs3739902 to be determined. Preferably, it allows the T allele of this marker to be detected; alternatively, it may enable the A allele to be detected. This marker is quite particularly preferred in the frame of the present invention.

According to another embodiment, the means for detecting the alleles of a SNP marker allows the allele of rs575916 to be determined. Preferably, it allows the G allele of this marker to be detected; alternatively, it may enable the C allele to be detected.

According to another embodiment, the means for detecting the alleles of a SNP marker allows the allele of rs365297 to be determined. Preferably, it allows the T allele of this marker to be detected; alternatively, it may enable the G allele to be detected.

The present invention also relates to a kit for the diagnosis of a predisposition to premature canities. Such a kit according to the invention contains several means for detecting in a sample of human genetic material, the alleles of at least two SNP markers of the human chromosome 9 selected from the markers rs306534, rs3739902, rs575916 and rs365297.

The kit such as described also contains a positive or negative control. By positive control is meant genetic material reflecting a predisposition to premature canities. By negative control is meant genetic material reflecting the absence of a predisposition to premature canities.

As specified in the preceding paragraphs, by means for detecting the alleles of a SNP marker is meant the nucleic acid probes which hybridise with only one of the alleles and not with the other under stringent conditions. Also disclosed is the use of all the primers which under certain conditions will make it possible to obtain products, obtained by PCR, of different sizes depending on the allele which is amplified. In this case, it is possible to detect simultaneously both alleles of a SNP, which indicates whether the individual is homozygous or heterozygous.

If probes are used, they are for example polynucleotide fragments corresponding to the region surrounding the SNP marker on the human chromosome 9. Such a fragment usually has a length included between 10 and 50 nucleotides, and preferably between 12 and 35 or 15 and 25 nucleotides. It may be a naturally occurring or synthetic fragment of DNA or RNA. The probe is advantageously immobilised on a support (a chip microarray).

The stringent conditions which can be used have already been explained in the preceding paragraphs.

The nucleic acid probe is advantageously linked to a detection agent, for example a radioactive, enzymatic, luminescent or fluorescent marker.

According to a preferred embodiment of a kit of the invention, the means for detecting the alleles of a SNP marker makes it possible to determine the allele of the marker rs306534.

Preferably, it enables the T allele of this marker to be detected; alternatively, the C allele of the marker can be detected.

According to another embodiment, the means for detecting the alleles of a SNP marker enable the allele of rs3739902 to be determined. This marker is a marker particularly preferred in the context of the invention. Preferably, the means mentioned enables the T allele of this marker to be detected; alternatively, it enables the A allele to be detected.

According to another embodiment, the means for detecting the alleles of a SNP marker enables the allele of rs575916 to be determined. Preferably, it enables the G allele of this marker to be detected; alternatively, it enables the C allele of the marker to be detected.

According to another embodiment, the means for detecting the alleles of a SNP marker enables the allele of the marker rs365297 to be determined. Preferably, it enables the T allele of this marker to be detected; alternatively, it enables the G allele of the marker to be detected.

It is also advantageous in a kit according to the invention to combine at least two means enabling the alleles of one of the four SNPs to be detected, for example two different probes, each allowing the C allele of the SNP rs306534 to be detected. According to another variant, the kit comprises several means enabling the alleles of at least two distinct SNPs of the SNPs rs306534, rs3739902, rs575916 and rs365297 to be detected, preferably means for detecting the alleles of 3 out of 4 SNPs or means for detecting the alleles of the 4 SNPs. Preferably, at least one of the means makes it possible to detect an allele of the SNP rs3739902.

It can also be envisaged that the kit comprises means making it possible to detect both alleles of a SNP selected from the SNPs rs306534, rs3739902, rs575916 and rs365297. For example, the kit may comprise a 1^{st} means enabling the T allele of rs3739902 to be detected and a 2^{nd} means enabling the A allele of this same SNP marker to be detected.

A kit according to the invention may also contain a combination of the different means mentioned in the preceding paragraphs. Preferentially, a kit according to the invention contains at least 3 different elements packaged together. It is also preferred that a kit of the invention contains less than 1000 different elements, preferentially less than 400.

According to a second aspect, the invention relates to the SNP markers rs3739902, rs2583805 and rs377090 of the human chromosome 9 identified by the inventors as forming a haplotype linked to predisposition to premature canities. These markers belong to the chromosomal region delimited on chromosome 9 by the microsatellite marker D9S290 and the telomeric region (telomer of the long arm) and are located within the DDX31 and GTF3C4 genes.

The inventors have in fact shown that certain alleles of these 3 markers are found in the individuals affected by premature canities with a frequency significantly higher than the normal frequency, which defines a haplotype linked to premature canities, designated HAP25-27.

The present invention covers a process for the diagnosis of a predisposition to premature canities in an individual. This diagnostic process comprises the determination of the alleles of the three SNP markers in a sample of genetic material of the said individual in order to identify the haplotype of the individual in relation to these three markers.

In order to diagnose a predisposition to premature canities in an individual or to confirm the diagnosis, it may prove to be advantageous to compare the haplotype HAP25-27 determined in said individual with the haplotype of other individual(s) serving as control(s), these individuals being obviously affected by premature canities or, conversely, being obviously not affected by premature canities. They may be in particular individuals more than 30 years old and not having conspicuous white hair.

It is also advantageous to select individuals as controls who are from the same geographical region as the individual to be diagnosed or who have a blood relationship with this individual, for example one of his/her parents or one of his/her siblings.

Certain haplotypes found with a significant frequency in the individuals affected by premature canities are in fact synonymous with a probable predisposition to premature canities in the individuals presenting these same haplotypes.

The present invention also relates to the use of means for detecting the alleles of the 3 markers rs3739902, rs2583805 and rs377090 defining the haplotype HAP25-27 for the diagnosis of a predisposition to premature canities wherein said means are nucleic acid probes. According to this use of the invention, the means make it possible to detect the alleles of the SNPs rs3739902, rs2583805 and rs377090 in a sample of genetic material of the individual who has to be diagnosed.

As specified in the preceding sections relating to the first aspect of the invention, for detecting the alleles of a SNP marker, sequencing devices (DNA or RNA), the primers for PCR and the nucleic acid probes which hybridise with only one of the alleles and not with the other under stringent conditions may be used. The three above-mentioned SNPs are indeed bi-allelic.

Such probes are, for example, polynucleotide fragments corresponding to the region surrounding the SNP marker on the human chromosome 9. Such a fragment usually has a length comprised between 10 and 50 nucleotides, preferably 12 to 35 nucleotide or 15 to 25 nucleotides. It may be a fragment of naturally occurring or synthetic DNA or RNA.

The probes are advantageously immobilised on a support (for example chip microarray).

The conditions of stringency applicable have already been explained in the preceding sections.

The nucleic acid probes are advantageously linked to a detection agent, for example a radioactive, enzymatic, luminescent or fluorescent marker. It may be advantageous if 3 distinct probes are used to determine the alleles of the 3 markers of the haplotype, to use 3 distinct detection agents, for example three fluorophores emitting at different wavelengths.

According to a preferred use, use is made of three different means in order to detect the allele of each of the 3 SNPs. Alternatively, use may be made of more than three different means, it is possible to use in particular at least two means for detecting one and the same allele of one of the SNPs or two means for detecting each of the alleles of one and the same SNP.

Every combination of the different means presented above can also be envisaged.

Preferably, the means used in the context of the invention make it possible to detect the T allele of the marker rs3739902, the G allele of the marker rs2583805 and the T allele of the marker rs377090.

It is important to note that every other combination can also be envisaged in the context of the present invention, for example means for detecting the T allele of the marker rs3739902, the C allele of the marker rs2583805 and the C allele of the marker rs377090. In fact, it may be for example as informative to detect the absence of the haplotype (T, G, T) as to detect the presence of the haplotype (T, C, C) in order to establish the diagnosis.

The present invention also relates to a kit for the diagnosis of a predisposition to premature canities. A kit according to the invention comprises means for determining the allelic form of the SNP markers rs3739902, rs2583805 and rs377090 in a sample of genetic material of an individual. These SNP markers are present on the human chromosome 9 and make it possible to define a particular haplotype, statistically linked to premature canities and hence reflecting a predisposition to premature canities in subjects still not showing any symptoms.

The kit such as described may also but not necessarily comprise a positive or negative control. By positive control is meant genetic material reflecting a predisposition to premature canities, for example a DNA sample from a person affected by premature canities. By negative control is meant genetic material reflecting the absence of a predisposition to premature canities. By definition, the means for detecting the alleles of the 4 markers present in the kit lead to a negative result when applied to the negative control, whereas they lead to a positive result when applied to the positive control.

By means for detecting the alleles of a SNP is meant the nucleic acid probes which hybridise with only one of the alleles and not with the other under stringent conditions.

Such probes are for example polynucleotide fragments corresponding to the regions flanking the SNPs markers on the human chromosome 9. Such fragments usually have a length included between 10 and 50 nucleotides, and preferably from 12 to 35 or from 15 to 25 nucleotides. They may be naturally occurring or synthetic DNA or RNA fragments. They are advantageously immobilised on a support (for example chip microarray).

The nucleic acid probe is advantageously linked to a detection agent for example a radioactive, enzymatic, luminescent or fluorescent marker.

It is also advantageous in a kit of the invention to combine more than 3 means making it possible to detect the alleles of three SNPs. For example, a kit according to the second aspect of the invention may contain for example two different probes, each making it possible to detect the T allele of the marker rs3739902.

It is can also be envisaged that the kit contains means making it possible to detect both alleles of one of the 3 SNPs, of 2 or even of all 3 SNPs. For example, the kit may contain a first means making it possible to detect the T allele of the marker rs3739902 and a second means making it possible to detect the A allele of this same marker and two other means making it possible to detect one of the two alleles of the markers rs2583805 and rs377090.

A kit according to the invention may also contain a combination of the different means mentioned in the preceding paragraphs. Preferentially, a kit according to the invention contains at least 3 different elements packaged together. It is also preferred that a kit of the invention contains less than 1000 different elements, preferentially less than 400.

It is also envisaged in the context of the present invention to scan the region of human chromosome 9 flanked by the SNP markers rs306534 and rs365297, for mutations (sequence variants) other than the polymorphisms illustrated by the 6 SNPs mentioned in the present invention. The example 4 is an illustration of this application in order to determine informative mutations. Such mutations found by a scan of the region between markers rs306534 and rs365297 on the human chromosome 9, for example the mutations disclosed in the Example 4, may advantageously be used as markers of the premature canities trait. Means for detecting said mutations may be used in the same manner as the means for detecting the alleles of the SNPs rs306534, rs3739902, rs575916 and rs365297 in the uses and processes of the invention.

According to a third aspect, the application of the results on the implication of polymorphisms within chromosome 9 for the predisposition to premature canities to the diagnosis of premature canities (or premature turning white of the hair) in other non-human mammals is described. In this situation the genetic diagnosis of premature canities is based on the information provided by the region of the genome of the said mammal which is homologous to the region of the human chromosome 9 flanked by the SNP markers rs306534 and rs365297.

According to this aspect, the use of at least one nucleotide fragment of at least 18 consecutive nucleotides the sequence of which corresponds to all or part of the chromosomal region of the genome of the said mammal, which is homologous to that of the human chromosome 9 flanked by the SNP markers rs306534 and rs365297 for the diagnosis of a predisposition to premature canities in this non-human mammal, is described.

This chromosomal region of the genome of the non-human mammal which is homologous to that of the human chromosome 9 flanked by the SNP markers rs306534 and rs365297 (limits included) will be designated "homologous region" in the present application.

According to this feature, the non-human mammal is preferably the horse, in order to diagnose a premature whitening of the horsehair.

Polynucleotide fragments having a minimum length of 18 nucleotides, the sequence of which corresponds at least in part to the homologous region and which make it possible to diagnose a predisposition to premature canities, are described. The polynucleotide fragment to which reference is made corresponds to a fragment of a chromosome. This fragment has a minimum length of 18 nucleotides and a maximum length which may extend to the total length of the homologous region in question. Preferably, the fragment has a number of nucleotides greater than 18. A particularly preferred length is comprised between 18 and 10,000 nucleotides, and preferably between 30 and 8,000 nucleotides.

As regards the chemical nature of this polynucleotide fragment, it may be a single- or double-stranded, circular or linear DNA molecule, an RNA molecule or any other molecule envisaged by the definition of polynucleotide fragment given above. It is preferably a molecule capable of interacting with the genetic material of the mammal to be diagnosed.

The polynucleotide fragment such as described may be naturally occurring or synthetic, or may be in part one and in part the other, in particular if it is a "duplex" molecule constituted of two strands of different origins. According to different cases envisaged, the polynucleotide fragment may have been isolated, it may have undergone a purification step. It may also be a recombinant fragment, for example synthesised in another organism. According to a preferred example, it is a DNA fragment that has been amplified by PCR (Polymerisation Chain Reaction), and then purified.

This use may consist in particular in determining the alleles of SNP markers present in the homologous region in the genetic material of the mammal to be diagnosed. Any extract from the body of the non-human mammal containing DNA of this mammal is suitable as genetic material. It may be in particular a blood sample, or skin cells or hairs.

The sample containing the genetic material may be a single drop of blood which is sufficient for the implementation of a process according to the invention. Samples of other body fluids may be used in the context of the invention. The use of a few cells derived from the mammal can also be envisaged.

According to other specific constructs envisaged, the first use makes use of a polynucleotide fragment associated with a probe. This characteristic makes it possible, among other things to monitor the localisation of the fragment, from the extracellular medium to the cell or from the cytoplasm to the nucleus or to specify its interaction with the DNA or RNA or proteins. The probe may also enable the degradation of the fragment to be monitored. The probe is preferably fluorescent, radioactive or enzymatic in nature. The specialist skilled in the art will know which probe is best suited depending on the characteristic that it is desired to be able to monitor.

The polynucleotide fragment, use of which is made in the context of this use, may be used in a hybridisation test, a sequencing, micro-sequencing or a mismatching detection test.

This fragment contains at least 18 consecutive nucleotides, these 18 nucleotides constituting a sequence which corresponds to all or part of the homologous sequence.

According to another particular case, the fragment described may correspond to one or more exons of a gene of the homologous region.

It is possible to use several polynucleotide fragments the sequence of which corresponds at least in part to all or part of the homologous region, for example two or three fragments having a distinct sequence or at least partially distinct.

### Legends to the Figures

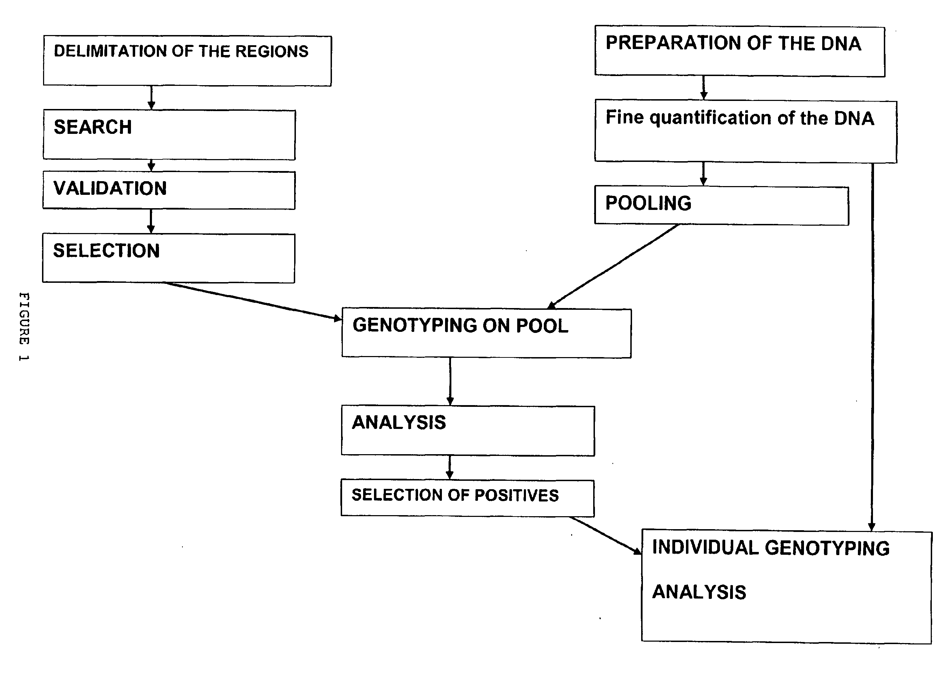

**Figure 1****:** Recapitulative flow chart of the different steps in the analysis of the B region with the aid of the technology based on the SNPs.

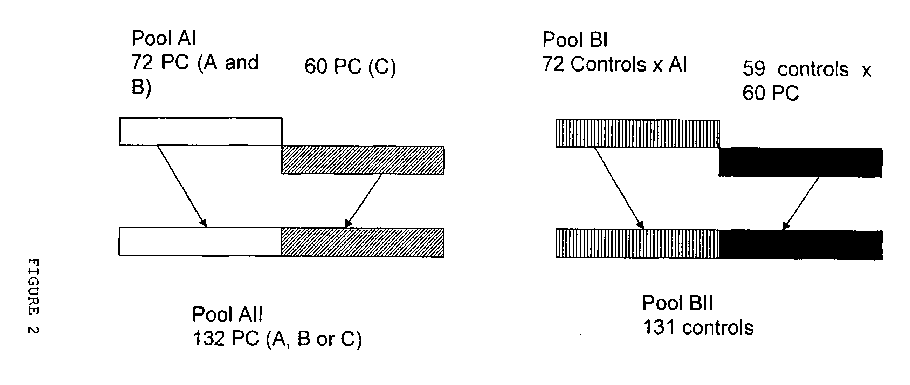

**Figure 2****:** Composition of the 4 pools. The pools Al and All are composed of individuals affected by premature canities. The two control pools Bl and BII are composed of individuals "crossed" with regard to origin and age with the individuals affected by premature canities.

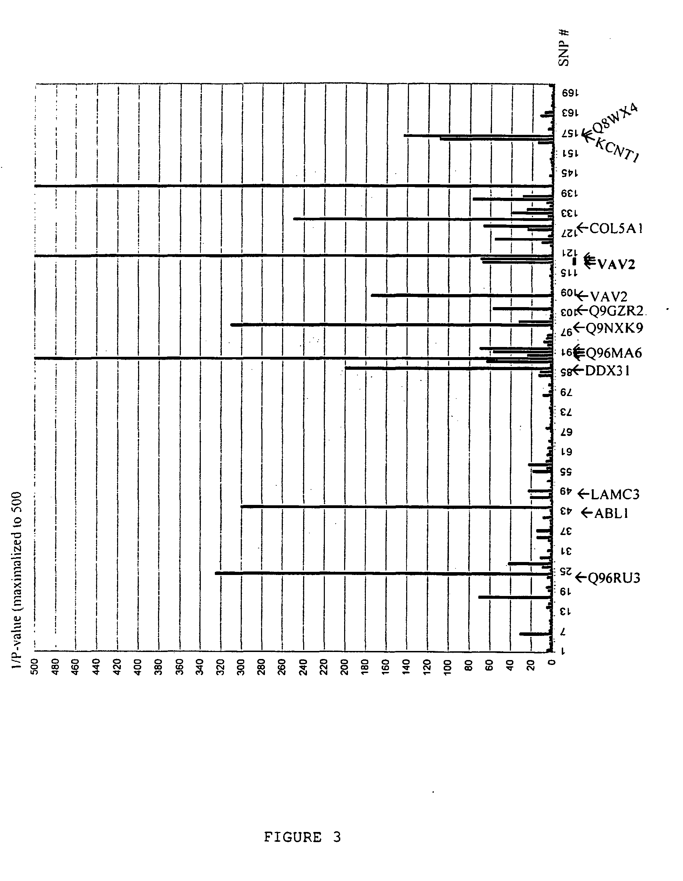

**Figure 3**: Graph indicating the significance of the 171 SNPs tested on the pools for the B region. The SNPs, numbered from 1 to 171 along the B region (from the telomer p towards the telomer q) are represented along the abscissa, each SNP being separated from its neighbours by a region of 30 kb on average. 1/p is represented along the ordinate, p being the statistical significance. However, the 1/p values greater than 500 (i.e. p<0.02) were maximised at 500.

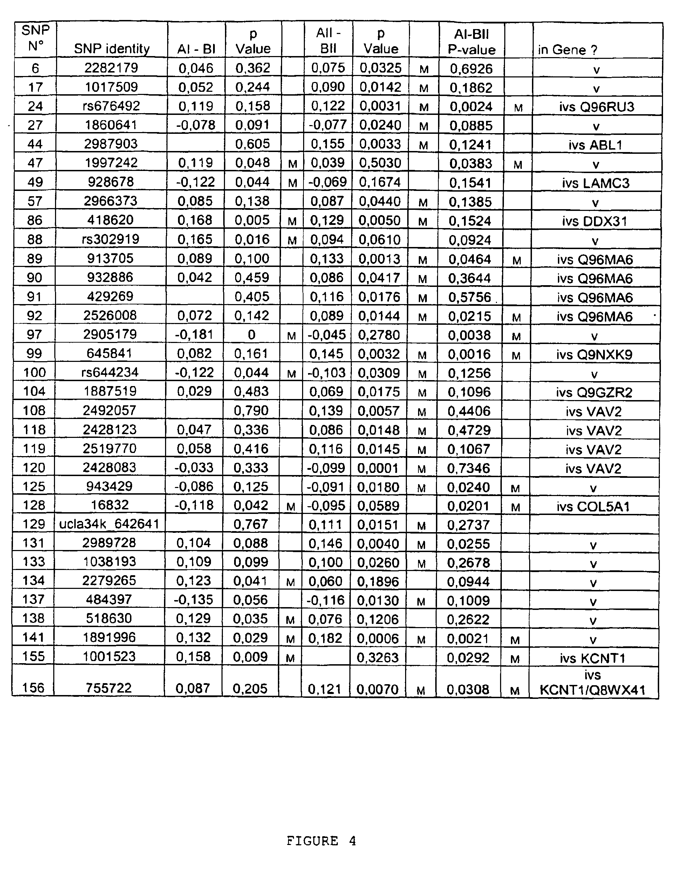

**Figure 4****:** Table listing the 33 SNPs selected for the individual genotyping. The first column indicates their number (number assigned in the previous step from 1 to 171 along the B region, from telomer p towards the telomer q). The second column indicates the identity of the SNP. The subsequent columns indicate the values of the different comparisons A-B (Al-Bl; All-Bll; Al-Bll) with the associated p value. The reference "M" signifies that the value of the significance "p" is less than 0.05. The last column specifies the gene possibly overlapped by the said SNP.

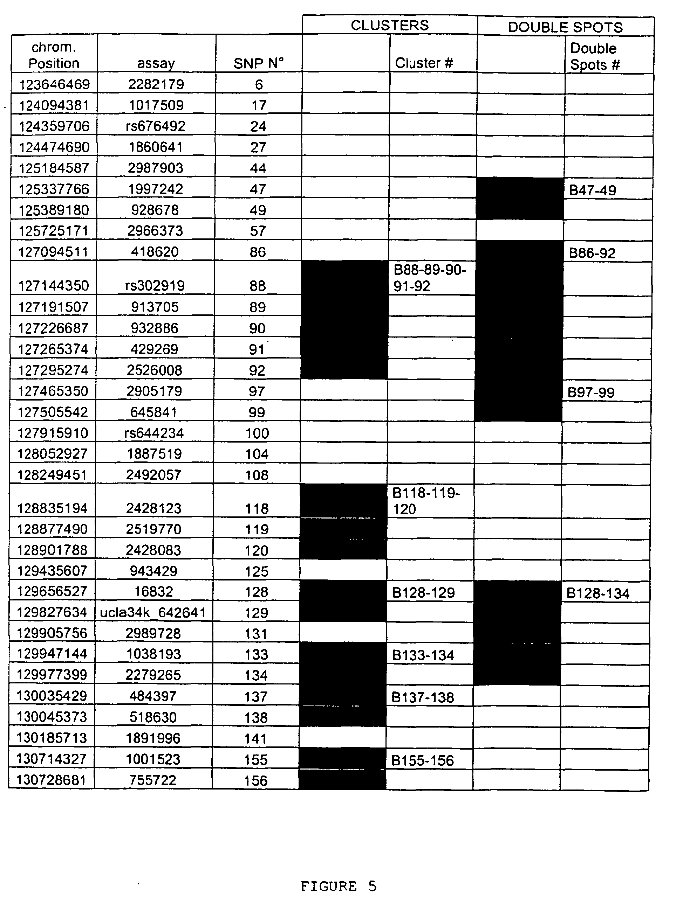

**Figure 5**: Table listing the 33 SNPs selected for the individual genotyping. The first column indicates the position on the chromosome, the second their identifier, the next column their number (number assigned in the previous step from 1 to 171 along the B region, from the telomer p towards the telomer q). The subsequent columns indicate whether the SNPs are present within a cluster or double spot.

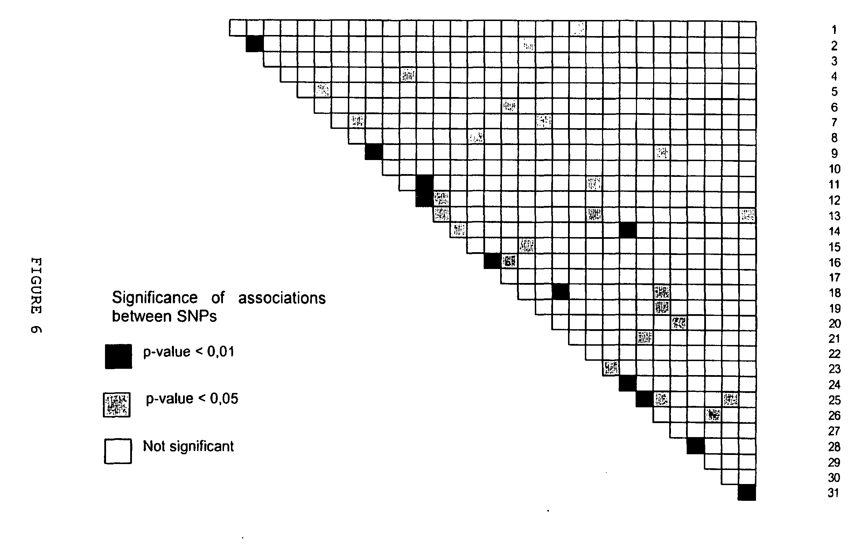

**Figure 6****:** Schematic representation of the results of linkage disequilibrium on the B region. The significance of the associations between SNPs taken two at a time is shown by a colour code.

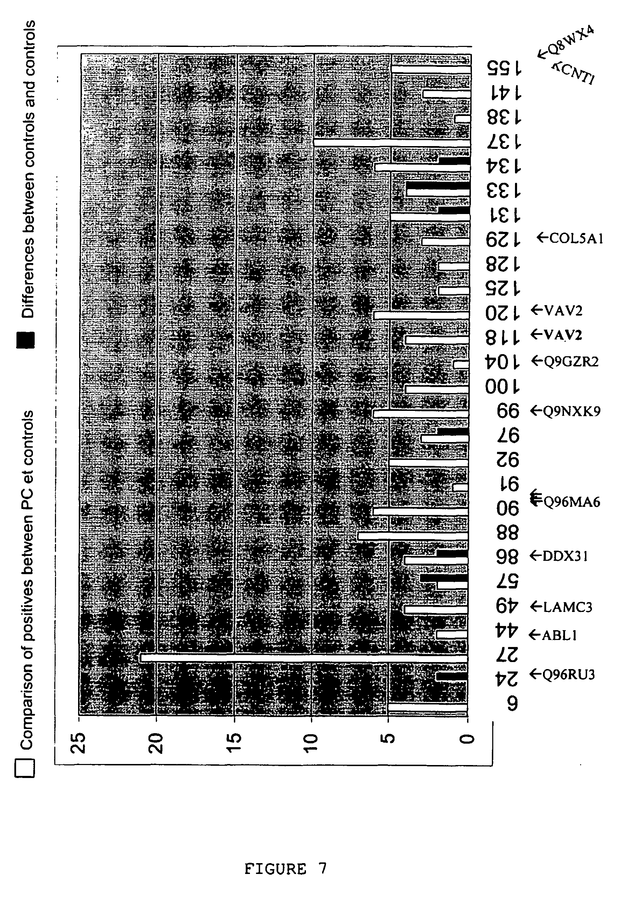

**Figure 7****:** Graph presenting the comparison of the allelic/genotypic frequencies for each SNP of the B region in the groups 'premature canities' and control, highlighting the SNPs/phenotype combinations. The genes concerned are indicated along the abscissa with the SNPs.

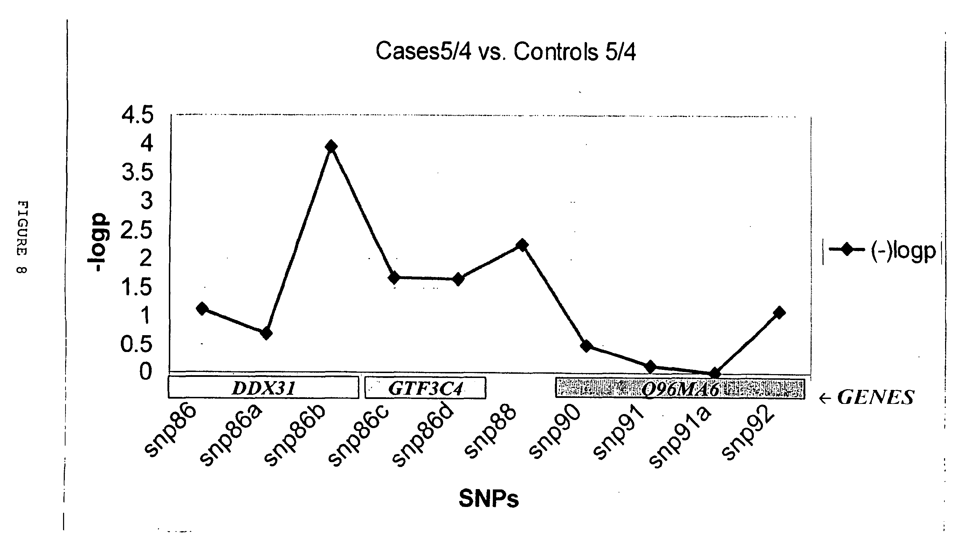

**Figure 8****:** Graph presenting the -logp value, p being the "p value" for the 10 SNPs used in a first step in the region of interest. The "p value" was obtained by comparison of the haplotype frequencies between the individuals affected presenting a score of 4 or 5 and the control individuals, corresponding to the individuals of the groups 4 and 5. The graph also shows the separation between the two haplotypes 86-88 and 90-92. The spacing between the SNPs on the abscissa axis is arbitrary and is not proportional to the inter-SNP distance. The genes within which the SNPs are located are also mentioned.

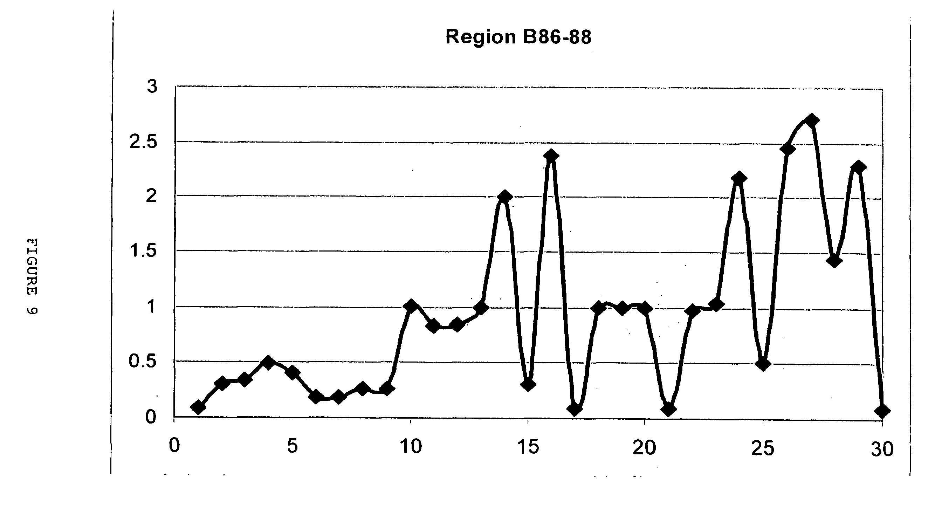

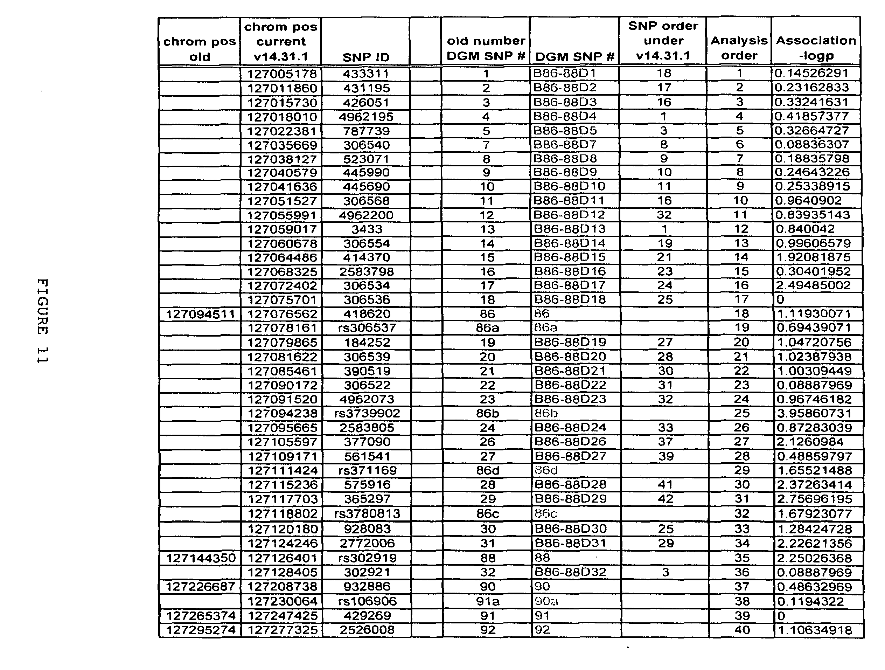

**Figure 9**: Graph presenting the -logp value, p being the "p value" for the 30 SNPs added in a second stage in the region of interest. The variables are the same as for figure 8. The number of the SNP from 1 to 30 for the 30 SNPs added is indicated along the abscissa. The correspondence between the number of the SNP (out of 30) and the identity of the SNP is explained in the table of figure 11 (old number DGM SNP#). Again, the abscissa X does not represent at scale the relative position of the SNP to each other.

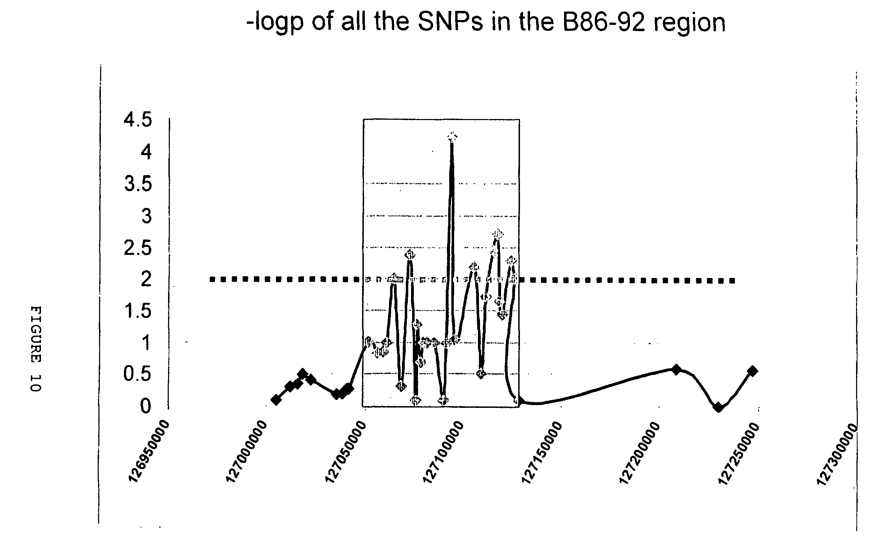

**Figure 10****:** Graph presenting the -logp value, p being the "p value" for the 40 SNPs (10+30) in the region of interest. The number of the SNP from 1 to 40 for the total of 40 SNPs examined is indicated along the abscissa. The correspondence between the number of the SNP (out of 40) and the identity of the SNP is explained in the table of figure 11, right part (analysis number). The scale of the X represents the relative position of the SNP to each other on the physical map of chromosome 9. The region where the linkage is most significant is indicated.
**Figure 11****:** Table listing the 40 SNPs examined in the region of interest (region B86-92). The first column indicates the position of some SNPs on the chromosome 9, according to the "Freeze of UCSC of December 2001" based on the Build NCBI 28 (hg 10 Dec2001 NCBI Build 28) whereas the second column indicates the position according to version V14.31.1 of the ENSEMBL sequences library which is based on the Build NCBI 31 (November 2002). The subsequent columns indicate respectively the GDB identifier of the SNP, the numbering of the SNP in the first phase of example 3 (10 + 30) and the numbering in the second phase (40) and finally the value of the association (-Iogp).
**Figure 12****:** This figure indicates for the 6 SNPs of the invention the adjacent sequence on chromosome 9, as well as the two alleles of the SNP that can be found.

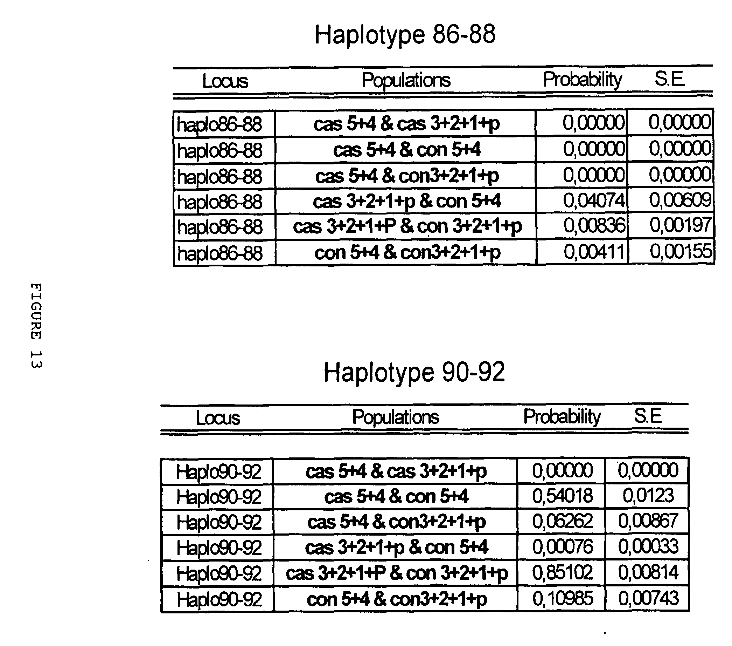

**Figure 13****:** The tables indicate the association values for the two haplotypes (S.E. means standard error). In fact, the SNP 86-88 and 90-92 are finally distributed in 2 regions in linkage disequilibrium.

### EXAMPLES

### EXAMPLE 1

In order to explore canities from a genetic point of view, a segregation study was performed of the DNA in families in which canities appears very early in life. In order to guarantee the best chances of success for this search for the genes, the composition of the sample for the study resulted from the application of a rigorous protocol for the assignment of the phenotype and the selection of the families. The premature canities (PC) phenotype was attributed only to the individuals who had white hair before they were 25 years old and half of whose scalp hair was grey at 30 years of age. The families were selected for the study on the basis of their statistical performances in the segregation analysis.

At the end of a series of preselections made on the basis of the statistical power of the sample and of the confirmation of the phenotypes, 12 families were selected to participate in a linkage study and DNA was prepared from a sample of peripheral blood taken from each of the informative individuals (presenting and not presenting the PC trait).

The study performed is described according to three principal periods:
Period 1: Determination of the potential of the study. A first selection of the most informative families is carried out by a linkage analysis simulation.
Period 2: Medical confirmation of the phenotypes and collection of blood samples from the preselected families. This verification campaign results in a new list of candidate families for the study. A new linkage simulation makes it possible to estimate the potential of the corrected sample.
Period 3: Global genetic linkage analysis of PC on the whole human genome. Familial segregation analysis of the DNA of the 22 autosomal chromosomes and the X chromosome in order to detect the regions which are linked to the PC trait.

From the set of analyses performed by fixing or not fixing parameters for the transmission of the PC, a potential locus emerges on chromosome 9 between the microsatellite marker D9S290 and the telomeric region (telomer of the long arm). The locus (chromosome 9q31-q32) shows suggestive signs of linkage to premature canities.

This study, and in particular the disagreement between the scores obtained for the parametric/non-parametric analyses suggests that premature canities is not caused by a small number of genes with a major effect, but is rather controlled by a multifactorial system including the action of several predisposing genes.

### EXAMPLE 2: Analysis of the region of interest with SNP 'Single Nucleotide Polymorphism'

Subsequent to the work presented in Example 1, the inventors continued the analysis of the region of chromosome 9 with the aid of the technology based on the SNPs in order to demonstrate the genes implicated in premature canities.

The SNPs (single nucleotide polymorphisms) represent a form of polymorphism which is particularly widespread in the human genome and very stable. The number of SNP is estimated to be about 1 SNP every 100 nucleotides, which makes it possible to construct a genuine map of the human genome with the aid of the SNPs. The SNPs are often classed in different categories, in particular depending on whether they are in a coding region or not, in a regulatory region or in another non-coding region of the genome, whether the polymorphism modifies the encoded amino acid or not, etc......

Since the conclusion of the "Human Genome Project" programme, the SNPs are now better known and referenced, as are their positions in the genome (GDB).

Different methods have been developed to detect these polymorphisms between different individuals, often based on the methods used for detecting point mutations (RFLP-PCR, hybridisation with specific oligomers of the alleles, mini-sequencing, direct sequencing......).

In the context of the present application, the inventors have used the MALDI-TOF (matrix-assisted laser desorption /ionization time-of-flight mass spectrometry) technology to detect the different alleles of the candidate SNPs. Further details concerning this technology are known to the specialist skilled in the art and are described in various publications (Stoerker J, et al. Nat. Biotechnol. 2000 Nov; 18 (11): 1213-6 and Tang K, et al., Proc. Natl. Acad. Sci. USA 1999 Aug. 96, 10016-20).

In a first phase, the inventors defined very precisely the region of chromosome 9 to be analysed with the SNPs. In a second step, about one thousand SNPs belonging to this region were pre-selected with respect to certain criteria (candidate SNPs in silico) and 232 were selected subsequent to an experimental validation step. In the next step, the inventors assembled the DNAs of the different individuals affected by premature canities and 'control' individuals in different groups, then performed the genotyping of these different groups by means of 167 SNPs selected from the 232. On conclusion of this genotyping the results made it possible to define 33 SNPs in order then to carry out an individual genotyping (and no longer on groups).

The different steps are described more fully in the following sections and are shown schematically in Figure 1.

### 1- Definition of the regions to be analysed by the SNPs

In a first step, the inventors defined more precisely the region of interest on chromosome 9, starting from the results obtained by means of the analysis with the microsatellite markers (see work described in Example 1) on the 12 families selected during that study.

The region on chromosome 9, designated "region B", is defined by its chromosomal position as well as by three other types of co-ordinates to give precision and optimal safety in the definition of this region for the subsequent steps.

Region B: chromosomal position 9q34.13-9q34.3 (qter)
Between the marker D9S290 and the telomer q
Between the SNP rs2096071 and the SNP rs1378955
Between the positions 123'405'258 bp* and 133'021'490 bp*
* = The position of the sequence (in terms of base pairs bp) is expressed as a function of the version of the data bank for the human genome updated on December 2001 (i.e. NCBI Build28).

### 2 - Search for candidate SNPs (in silico) and (experimental) validation.

Starting from the B region as defined above, a 2^{nd} step consisted in determining a collection of SNPs belonging to this region so as to obtain a map of markers of the region. These markers were also defined such that they cover the total length of the region in a homogeneous manner, equidistantly spaced from each other. The distance between the different SNPs was fixed at 30 kb on average. This operation was performed by the selection of almost one thousand SNPs meeting these criteria (candidate SNPs in silico).

Of these SNPs thus pre-selected during the first step, about 90% of the SNPs proved to be operational. By operational is meant that they can be amplified with the aid of the usual reagents. The selected SNPs were analysed in 92 control individuals (individuals of the Centre for the Study of Human Polymorphism) in order to validate the presence of at least two alleles for each of the SNP (validation of the polymorphism).

At the conclusion of this experimental selection, only the SNPs exhibiting an allelic frequency of the rarer allele of at least 10% were selected. By means of this method 232 SNPs were validated in the B region.

### 3 - Collection of the DNA (Pooling)

In order to increase the genotyping capacity, a pooling strategy was carried out on the DNA. The power of this method has been reported in various publications (in particular Werner et al., Hum. Mutat. 2002 Jul.; 20 (1): 57-64, Bansal et al., Proc. Natl. Acad. Sci. USA 2002 Dec 24; 99(26):16871-4).

In order to carry out this pooling, the DNAs of the different individuals with the 'premature canities' (PC) trait and that of the control individuals were pooled. This pooling was done such that each of the DNAs is represented in an equimolar manner in order to guarantee that no individual has a preponderating influence on the result with respect to another. For this purpose, the exact concentration of each of the DNA was measured by the "Picogreen" method in the different samples taken from the individuals.

Groups were constituted by taking into account a "phenotypic score of canities intensity" which was assigned to each individual in the following manner.

In a first step, two kinds of criteria were defined, the primary criteria to which were assigned score values of 2 and the secondary criteria to which were assigned score values of 1.

There are 2 primary criteria (score value=2 for each of them) which are: (i) first white hair before the age of 18 years; (ii) light salt-and-pepper scalp hair at the age of 30 years.

There are 3 secondary criteria (score value=1 for each of them) which are: (i) first white hair before the age of 25 years; (ii) dark salt-and-pepper scalp hair at 30 years; (iii) evidence in the family of premature canities.

By adding for each individual the scores obtained for each of the diagnostic criteria, it is possible to define for each individual a score of phenotypic intensity of premature canities. In this way it was possible to define several different groups according to the phenotypic score. Of the individuals affected, 72 whose score is higher than or equal to 4 or 5 and 132 individuals whose phenotypic score is higher than or equal to 2.

Group Al: this group is constituted by the DNA of the 72 PC individuals whose phenotypic score is 4 or 5.

Group All: this group is constituted by the DNA of the 132 PC individuals whose phenotypic score is 2, 3, 4 or 5.

Groups BI and BII: these groups are constituted by the DNA of the control individuals whose geographic origin is close to that of the PC individuals. For these control individuals, the selection criteria were: (i) an age over 40 years; (ii) the absence of a sign of canities in the control individual; (iii) the absence of evidence in the family of canities. The matching criteria with an individual of the group Al or All are an identical geographic origin, the same sex and an identical hair colour at 18 years.

in this way, except for the matching affected versus not affected by the phenotypic trait (PC), each PC individual of the group Al is represented by a control individual in group BI whose geographic place of origin is close or identical. The same holds for each individual of group All.

The constitution of the different groups is represented schematically in Figure 2.

The use of these rigorous methods of clinical diagnosis for affected subjects and control subjects give a guarantee of reliability concerning the quality of the phenotypic data. Moreover, the rigor of the matching according to the rules fixed by the inventors is the guarantee of the relevance of the statistical analysis comparing the genomic data derived from these individuals whether they are collected in pools or compared individually.

### 4 - Selection of the SNP validated for the genotyping on the grouped/pooled DNA.

167 SNPs were selected out of the 232 validated in step 2 during a new selection step. This new selection is based on the interval between the SNPs, fixed on average between 30 and 50 kb.

The different SNPs used for the successive steps are listed in the following tables. These tables also include 4 additional SNPs which were added in a subsequent step to complete the list. These 4 additional SNPs are the SNPs Nos 86, 97, 131 and 137.

The 171 SNPs of the B region were numbered in increasing order along (telomer p towards telomer q) the B region which they cover in a quasi-equidistant and homogeneous manner.

### Region B :

| **SNP N°** | **Identifier (GDB)** | **SNP N°** | **Identifier (GDB)** | **SNP N°** | **Identifier (GDB)** | **SNP N°** | **Identifier (GDB)** |
|---|---|---|---|---|---|---|---|
| 1 | 2096071 | 44 | 2987903 | 87 | 787469 | 130 | 2989736 |
| 2 | 2282394 | 45 | 2314027 | 88 | rs302919 | 131 | 2989728 |
| 3 | 2805103 | 46 | 1544012 | 89 | 913705 | 132 | 3012797 |
| 4 | 1331336 | 47 | 1997242 | 90 | 932886 | 133 | 1038193 |
| 5 | 1533967 | 48 | 928677 | 91 | 429269 | 134 | 2279265 |
| 6 | 2282179 | 49 | 928678 | 92 | 2526008 | 135 | 964138 |
| 7 | 2011978 | 50 | 2315073 | 93 | 2072058 | 136 | 515078 |
| 8 | 955910 | 51 | 933093 | 94 | rs739441 | 137 | 484397 |
| 9 | 1147360 | 52 | 2315076 | 95 | 2905078 | 138 | 518630 |
| 10 | rs940373 | 53 | 2315078 | 96 | 64967 | 139 | 752835 |
| 11 | 2498905 | 54 | 981759 | 97 | 2905179 | 140 | 1778993 |
| 12 | 2542248 | 55 | 2483469 | 98 | rs649168 | 141 | 1891996 |
| 13 | 1220653 | 56 | 2478858 | 99 | 645841 | 142 | 1106256 |
| 14 | 1867099 | 57 | 2966373 | 100 | rs644234 | 143 | 2382867 |
| 15 | ucla34k_454177 | 58 | 540621 | 101 | 532861 | 144 | 2065385 |
| 16 | 2241271 | 59 | 2994056 | 102 | 59071 | 145 | 872667 |
| 17 | 1017509 | 60 | 2275500 | 103 | 1179040 | 146 | 914400 |
| 18 | rs1182 | 61 | 10K-56700 | 104 | 1887519 | 147 | ucla34k_923462 |
| 19 | rs732074 | 62 | rs943851 | 105 | 1179001 | 148 | 1412512 |
| 20 | rs1125962 | 63 | 2282006 | 106 | ucla34k_576465 | 149 | rs968569 |
| 21 | ucla34k_598296 | 64 | 1887786 | 107 | 954052 | 150 | 210086 |
| 22 | 1322671 | 65 | 2076 | 108 | 2492057 | 151 | 783770 |
| 23 | 1570381 | 66 | 928013 | 109 | 2506715 | 152 | 872006 |
| 24 | rs676492 | 67 | 869381 | 110 | 2506696 | 153 | 1537414 |
| 25 | 2286792 | 68 | 3012757 | 111 | 1079783 | 154 | 574840 |
| 26 | 53558 | 69 | 2987378 | 112 | rs77905 | 155 | 1001523 |
| 27 | 1860641 | 70 | 3012717 | 113 | 129891 | 156 | 755722 |
| 28 | 885345 | 71 | 1331631 | 114 | 2027963 | 157 | 1318383 |
| 29 | rs1043368 | 72 | 1412075 | 115 | 628936 | 158 | 730399 |
| 30 | 1557126 | 73 | 1331625 | 116 | rs602990 | 159 | 1009473 |
| 31 | 947507 | 74 | 2149171 | 117 | 2428091 | 160 | 47713 |
| 32 | 914977 | 75 | ucla34k_694625 | 118 | 2428123 | 161 | 2297690 |
| 33 | 2210623 | 76 | 2296868 | 119 | 2519770 | 162 | 2139881 |
| 34 | 1475731 | 77 | rs1185193 | 120 | 2428083 | 163 | 1335099 |
| 35 | 928518 | 78 | 10K-52978 | 121 | 2789861 | 164 | 55096 |
| 36 | 1864709 | 79 | 563521 | 122 | 414848 | 165 | 2501566 |
| 37 | 944605 | 80 | 507998 | 123 | 1536474 | 166 | 2501559 |
| 38 | 2304812 | 81 | 2362369 | 124 | 943435 | 167 | 2183138 |
| 39 | 1866974 | 82 | 577416 | 125 | 943429 | 168 | 1054864 |
| 40 | 2269337 | 83 | 944812 | 126 | 2182640 | 169 | 2275781 |
| 41 | 2583839 | 84 | rs1470190 | 127 | ucla34k_177347 | 170 | 1891629 |
| 42 | 2791743 | 85 | 2247393 | 128 | 16832 | 171 | 1099298 |
| 43 | 2855181 | 86 | 418620 | 129 | ucla34k 642641 | | |

### 5 - Genotyping of the pooled DNA

In the case of the 171 SNPs selected at step 4, the next step was to determine their allelotype, i.e. the frequency of each of the alleles, and to do this for the 4 groups of DNA pooled in accordance with the severity and prematurity of the phenotype (see the definition of the 4 groups at stage 3 and Figure 2).

The allelic frequency of both alleles was determined for each of the SNPs in the four groups. The statistical significance of the standard deviations of the allelic frequencies between the groups Al and Bl or All and Bll is estimated by the "p" value representing the significance. The smaller the value of p the greater the statistical significance of the standard deviation.

The experiments were reproduced 3 times (3 PCR), each of the three PCR then being tested 5 times on the MALDI-TOF in order to obtain a reliable mean value.

Figure 3 illustrates the results obtained on the B region for each SNP (numbered from 1 to 171 along the B region). The ordinate represents 1/p, but the values greater than 500 (i.e. p<0.002) are maximised at 500.

**Table 1 summarises the results obtained:**

| Chromosome 9 | | |
|---|---|---|
| Al-Bl<0,05 **AND** All-Bll<0,05 | Al-Bl<0,05 | All-Bll<0,05 |
| 2 | 9 | 22 |

### Table 1: Genotyping of the pools, number of positive SNPs

These results demonstrate the existence of clusters, i.e. at least three consecutive SNPs (hence physically close to each other in the human genome) which all have a significance p less than 0.05 (called "positive SNP"). Some of these clusters are illustrated in Figure 3.

Table 2 recapitulates the different particularities in the distribution of the SNPs in the B region.

**Table 2: Particularities of the distribution of the positive SNPs in the B region.**

| Chromosome 9 | |
|---|---|
| Clusters (3 or more consecutive positive SNPs) | 2 |
| Pairs (2 consecutive positive SNPs) | 4 |
| 'Double spots' (2 positive SNPs separated by a negative SNP) | 2 |

The different genes of the B region which are detected by positive SNPs distributed in clusters, isolated or distributed as double 'spots' constitute a first series of candidate genes, including the predicted genes. The following is a list of them:

### B Region:

DDX31, GTF3C4, C9ORF9, TSC1, ABL1, LOC57109, FREQ, ADAMTS13, LAMC3, SURF5, SURF6, FCN2, FCN1, OLFM1, VAV2, ABO, CELL, SARDH.

A more detailed analysis was performed which made it possible to develop a new list of genes overlapped by a positive SNP by means of ENSEMBL (ENSEMBL v.8.30a.1 17 sep 2002). This list comprises the overlapped genes (coding, UnTranslated Region UTR, and intron) by a positive SNP, to the exclusion of the genes which are close to a positive SNP located in a regulatory region.

### Introns :

### Q96RU3, ABL1, LAMC3, Q96MA6, Q9NXK9, Q9GZR2, VAV2, COL5A1, KCNT1, Q8WX41

A new analysis for the predicted genes by means of ENSEMBL gave the following results :

| | | | |
|---|---|---|---|
| ENST00000298489, | ENST00000266097, | ENST00000263612, | ENST00000245590, |
| ENST00000298545, | ENST00000298546, | ENST00000298552, | ENST00000298554, |
| ENST00000298555, | ENST00000277434, | ENST00000277433, | ENST00000298632, |
| ENST00000291687, | ENST00000298656, | ENST00000298658, | ENST00000298660, |
| ENST00000277355, | ENST00000298678, | ENST00000298676, | ENST00000298656, |
| ENST00000298658, | ENST00000298660, | ENST00000277355, | ENST00000298678, |
| ENST00000298676, | ENST00000298682, | ENST00000298683, | ENST00000291744, |
| ENST00000291741, | ENST00000223427, | ENST00000198253, | ENST00000277527, |
| ENST00000263604, | ENST00000266109, | ENST00000298467, | ENST00000266100, |
| ENST00000277422, | ENST00000263609. | | |

The following tables list the predicted genes of the B region in the clusters, the double spots (DS) and the individual positive SNPs starting from the version NCBI Build 28 (Dec. 2001). "CDS" indicates coding sequence and "tx" transcript.

### REGION B

| SNP# | NAME | chrom | cdsStart | cdsEnd | txStart | txEnd | Strand | No. EXONS |
|---|---|---|---|---|---|---|---|---|
| 47 à 49 DS | | | | | | | | |
| | NAME | chrom | cdsStart | cdsEnd | txStart | txEnd | Strand | No. EXONS |
| | ENST00000298489 | chr9 | 125457741 | 125470257 | 125373136 | 125470567 | + | 28 |
| | ENST00000266097 | chr9 | 125373234 | 125470257 | 125373136 | 125470567 | + | 28 |
| | | | | | | | | |

| 86 à 92 | | | | | | | | |
|---|---|---|---|---|---|---|---|---|
| | NAME | chrom | cdsStart | cdsEnd | txStart | txEnd | Strand | No. EXONS |
| | ENST00000263612 | chr9 | 127045062 | 127120443 | 127044482 | 127120595 | - | 20 |
| | ENST00000245590 | chr9 | 127120792 | 127139136 | 127120534 | 127139622 | + | 5 |
| | ENST00000298545 | chr9 | 127175884 | 127328449 | 127175884 | 127328449 | - | 13 |
| | | | | | | | | |

| 97 à 99 | | | | | | | | |
|---|---|---|---|---|---|---|---|---|
| | NAME | chrom | cdsStart | cdsEnd | txStart | txEnd | Strand | No. EXONS |
| | ENST00000298555 | chr9 | 127469679 | 127471065 | 127469615 | 127471372 | + | 1 |
| | ENST00000277434 | chr9 | 127501047 | 127508171 | 127501047 | 127508171 | + | 8 |
| | ENST00000277433 | chr9 | 127481205 | 127508171 | 127480906 | 127508695 | + | 11 |
| | | | | | | | | |

| 86 à 99 DS chr9:127094511-127SOSS42 | | | | | | | | |
|---|---|---|---|---|---|---|---|---|
| | NAME | chrom | cdsStart | cdsEnd | txStart | txEnd | Strand | No. EXONS |
| | ENST00000263612 | chr9 | 127045062 | 127120443 | 127044482 | 127120595 | - | 20 |
| | ENST00000245590 | chr9 | 127120792 | 127139136 | 127120534 | 127139622 | + | 5 |
| | ENST00000298545 | chr9 | 127175884 | 127328449 | 127175884 | 127328449 | - | 13 |
| | ENST00000298546 | chr9 | 127334141 | 127338631 | 127328556 | 127340224 | + | 4 |
| | ENST00000298552 | chr9 | 127346431 | 127379066 | 127341543 | 127394815 | - | 23 |
| | ENST00000298554 | chr9 | 127436872 | 127441241 | 127436872 | 127441244 | + | 6 |
| | ENST00000298555 | chr9 | 127469679 | 127471065 | 127469615 | 127471372 | + | 1 |
| | ENST00000277434 | chr9 | 127501047 | 127508171 | 127501047 | 127508171 | + | 8 |
| | ENST00000277433 | chr9 | 127481205 | 127508111 | 127480906 | 127508695 | + | 11 |
| | | | | | | | | |

| 118 à 120 | | | | | | | | |
|---|---|---|---|---|---|---|---|---|
| | NAME | chrom | cdsStart | cdsEnd | txStart | txEnd | Strand | No. EXONS |
| | ENST00000298632 | chr9 | 128877580 | 128878579 | 128877580 | 128878579 | - | 1 |
| | ENST00000291687 | chr9 | 128750384 | 128978689 | 128750384 | 128978689 | - | 27 |
| | | | | | | | | |

| 128 a 129 chr9:129656527-129827634 | | | | | | | | |
|---|---|---|---|---|---|---|---|---|
| | NAME | chrom | cdsStart | cdsEnd | txStart | txEnd | Strand | No. EXONS |
| | ENST00000298656 | chr9 | 129757553 | 129770881 | 129757553 | 129770881 | - | 16 |
| | ENST00000298658 | chr9 | 129757607 | 129781523 | 129757607 | 129781523 | - | 13 |
| | ENST00000298660 | chr9 | 129757553 | 129786638 | 129757553 | 129786638 | - | 26 |
| | ENST00000277355 | chr9 | 129607564 | 129789067 | 129607564 | 129789067 | + | 29 |
| | ENST00000298678 | chr9 | 129811215 | 129812613 | 129811213 | 129812613 | + | 2 |
| | ENST00000298676 | chr9 | 129814180 | 129826506 | 129607438 | 129826534 | + | 37 |
| | | | | | | | | |

| 133 à 134 chr9:129947144-129977399 | | | | | | | | |
|---|---|---|---|---|---|---|---|---|
| | 0 | | | | | | | |
| | | | | | | | | |

| 137 à 138 chr9:130035429-130045373 | | | | | | | | |
|---|---|---|---|---|---|---|---|---|
| | 0 | | | | | | | |
| | | | | | | | | |

| 128 à 134 DS chr9:129656527-129977399 | | | | | | | | |
|---|---|---|---|---|---|---|---|---|
| | NOM | chrom | cdsStart | cdsEnd | txStart | txEnd | Strand | No. EXONS |
| | ENST00000298656 | chr9 | 129757553 | 129770881 | 129757553 | 129770881 | - | 16 |
| | ENST00000298658 | chr9 | 129757607 | 129781523 | 129757607 | 129781523 | - | 13 |
| | ENST00000298660 | chr9 | 129757553 | 129786638 | 129757553 | 129786638 | - | 26 |
| | ENST00000277355 | chr9 | 129607564 | 129789067 | 129607564 | 129789067 | + | 29 |
| | ENST00000298678 | chr9 | 129811215 | 129812613 | 129811213 | 129812613 | + | 2 |
| | ENST00000298676 | chr9 | 129814180 | 129826506 | 129607438 | 129826534 | + | 37 |
| | ENST00000298682 | chr9 | 129864608 | 129868564 | 129864598 | 129870351 | + | 5 |
| | ENST00000298683 | chr9 | 129864608 | 129869270 | 129864598 | 129871307 | + | 7 |
| | ENST00000291744 | chr9 | 129864608 | 129871199 | 129864598 | 129871307 | + | 8 |
| | ENST00000291741 | chr9 | 129864608 | 129871199 | 129864598 | 129871307 | + | 7 |
| | ENST00000223427 | chr9 | 129893584 | 129901655 | 129893369 | 129901747 | - | 9 |
| | ENST00000198253 | chr9 | 129896270 | 129901655 | 129893369 | 129901747 | - | 8 |
| | | | | | | | | |

| 155 à 156 chr9:130714327-130728681 | | | | | | | | |
|---|---|---|---|---|---|---|---|---|
| | NOM | chrom | cdsStart | cdsEnd | txStart | txEnd | Strand | No. EXONS |
| | ENST00000277527 | chr9 | 130609471 | 130715656 | 130609471 | 130715656 | - | 4 |
| | ENST00000263604 | chr9 | 130691065 | 130775279 | 130691064 | 130775281 | + | 29 |
| | | | | | | | | |
| | individuals positive SNPs | | | | | | | |
| | | | | | | | | |
| 6 | | | | | | | | |
| | no genes | | | | | | | |
| | | | | | | | | |
| 17 | | | | | | | | |
| | no genes | | | | | | | |
| | | | | | | | | |
| 24 | | | | | | | | |
| | NAME | chrom | cdsStart | cdsEnd | txStart | txEnd | Strand | No. EXONS |
| | ENST00000266109 | chr9 | 124213577 | 124360885 | 124213576 | 124360901 | - | 15 |
| | | | | | | | | |
| 27 | | | | | | | | |
| | no genes | | | | | | | |
| 44 | | | | | | | | |
| | NAME | chrom | cdsStart | cdsEnd | txStart | txEnd | Strand | No. EXONS |
| | ENST00000298467 | chr9 | 125063030 | 125234391 | 125063030 | 125234391 | + | 11 |
| | ENST00000266100 | chr9 | 125184157 | 125234391 | 125183776 | 125236384 | + | 11 |
| | | | | | | | | |
| 57 | | | | | | | | |
| | no genes | | | | | | | |
| | | | | | | | | |
| 100 | | | | | | | | |
| | no genes | | | | | | | |
| | | | | | | | | |
| 104 | | | | | | | | |
| | NAME | chrom | cdsStart | cdsEnd | txStart | txEnd | Strand | No. EXONS |
| | ENST00000277422 | chr9 | 128045772 | 128056657 | 128044878 | 128056857 | - | 8 |
| | | | | | | | | |
| 108 | | | | | | | | |
| | no genes | | | | | | | |
| | | | | | | | | |
| 125 | | | | | | | | |
| | NAME | chrom | cdsStart | cdsEnd | txStart | txEnd | Strand | No. EXONS |
| | ENST00000263609 | chr9 | 129380168 | 129507477 | 129380168 | 129507477 | + | 9 |
| | | | | | | | | |
| 141 | | | | | | | | |
| | no genes | | | | | | | |

### 6 - Selection of the SNPs for the genotyping of the individual DNAs.

Of the 171 SNPs used for the genotyping on the pooled DNAs, 33 were selected for the genotyping of the individual DNAs. The SNPs selected do in fact show a statistically significant deviation when the genotyping is done on the pools, i.e. p<0.05 for AI-BI, All-BII or AI-BII.

The list of the SNPs thus chosen and the A-B comparison are given in Figure 4. Table 3 summarises the results obtained.

**Table 3: Choice of the positive SNPs (total 33) following the genotyping results on the pools.**

| Chromosome 9 | | | |
|---|---|---|---|
| Al-Bl<0,05 **AND** All-Bll<0,05 | Al-Bl<0,05 | All-Bll<0,05 | Al-BII<0,05 |
| 3 | 11 | 25 | 11 |

The choice of the 33 SNPs for the individual genotyping is concentrated on the SNPs present in the clusters, those forming pairs (2 positive consecutive SNPs) and those forming 'double spots' (2 positive SNPs separated by a negative SNP). Figure 5 illustrates the distribution of the 33 SNPs selected.

In fact, it was observed that the estimation of the allelic frequencies on pools (and not on individuals) can lead to 'false' positives and that this tendency is increased when the pools contain less than 200 DNA. As a result the isolated positive SNPs were in part eliminated as well as those being inconsistent with the controls (BI and BII).

The 33 SNPs were analysed individually on all of the available DNAs (187 individuals with the PC phenotype and 186 control individuals without PC phenotype).

Of the 33 SNPs selected, 16 SNPs are in clusters, 6 SNPs are in double spots and 12 SNPs are individually positive (isolated positive SNP).

This individual genotyping makes it possible to calculate precisely the frequency of the alleles and the genotypes observed in the different groups. This set of data also makes it possible to compare the distribution of the haplotypes observed at the level of the positive SNPs organised in 'clusters'. By haplotype is meant the combination of alleles tending to be transmitted together.

The integrated analysis of this set of data makes it possible to determine the SNPs or groups of SNPs which show an association with the PC trait, i.e. an allele or a set of alleles which, in a population, are transmitted more frequently with this trait.

### 7 - Study of the linkage disequilibrium (LD)

The linkage disequilibrium was analysed by means of the GenePop programme, in the absence of data concerning the phase of the haplotypes on the chromosomes analysed.

The linkage disequilibrium is a situation in which 2 genes (alleles) segregate together at a higher frequency than the frequency predicted by the product of their individual frequencies. That means that the two genes are not independent because they segregate together more frequently than anticipated statistically, there is thus a deficit of independence between alleles situated close to each other on the same chromosome.

This analysis of linkage disequilibrium has made it possible to define blocks of DNA which are represented by several markers, the co-segregation of the alleles of which deviates from a co-segregation determined by chance alone. This situation is produced by an absence or deficit of recombination within this block. The size of the regions exhibiting a linkage disequilibrium varies according to the chromosomal regions, it seems to range from 10 kb to 200 kb. The results are presented in Figure 6.

### 8 - Comparison of the allelic/genotypic frequencies for each SNP.

This comparison of the allelic/genotypic frequencies was carried out for each SNP in the premature canities (1 to 5) and in the control groups. The results obtained are reproduced in the following tables and presented in Figure 7.

The column "con-con" indicates the comparison between the different groups of control individuals. The column "aff" indicates the comparisons for each group of persons affected against all of the other groups of affected or control persons.

| **SNP** | **Con-con** | **aff** | **counts** | **SNP** | **Con-con** | **aff** | **counts** |
|---|---|---|---|---|---|---|---|
| 6 | 0 | 5 | 5 | 104 | 0 | 1 | 1 |
| 24 | 2 | 0 | 2 | 118 | 0 | 4 | 4 |
| 27 | 0 | 21 | 21 | 120 | 0 | 6 | 6 |
| 44 | 0 | 2 | 2 | 125 | 0 | 2 | 2 |
| 49 | 0 | 4 | 4 | 128 | 0 | 2 | 2 |
| 57 | 3 | 2 | 5 | 129 | 0 | 3 | 3 |
| 86 | 2 | 4 | 6 | 131 | 2 | 5 | 7 |
| 88 | 0 | 7 | 7 | 133 | 4 | 4 | 8 |
| 90 | 0 | 6 | 6 | 134 | 2 | 6 | 8 |
| 91 | 0 | 1 | 1 | 137 | 0 | 10 | 10 |
| 92 | 0 | 5 | 5 | 138 | 0 | 1 | 1 |
| 97 | 2 | 3 | 5 | 141 | 0 | 3 | 3 |
| 99 | 0 | 6 | 6 | 155 | 0 | 5 | 5 |
| 100 | 0 | 4 | 4 | | | | |

### 9 - Conclusions.

The principal conclusions which may be drawn from the results are the following:
Firstly, there is a great similarity between the observations made on the analysis of the pools and the individual genotypings.
The large "clusters" are confirmed.
The B region reveals an interval in linkage disequilibrium (major cluster) which is strongly associated with the premature canities trait (SNP 418620 to SNP 2526008, position 126'544'533 nt to position 126'745'296 nt, i.e. a size of 201 kb). This cluster includes the genes DDX31, GTF3C4 and Q96MA6.
The genes or predicted genes identified in the intervals associated with a positive haplotype or a cluster of positive SNPs are the following:

### Haplotype 27

> FREQ
   PubMed on Product: frequenin homolog/ Mouse Ortholog: Freq
   Start (position on chrom.): 124490317 End (position on chrom.): 124554366
> NT_030046.18
   Start (position on chrom.): 124458070 End (position on chrom.): 124489558
> NT_030046.17
   Start (position on chrom.): 124371672 End (position on chrom.): 124452860

### Haplotype 97-100

> GTF3C5
   PubMed on Product: general transcription factor IIIC, polypeptide 5
   Start (position on chrom.): 127480920 End (position on chrom.): 127508694
> CEL
   PubMed on Product: carboxyl ester lipase (bile salt-stimulated)
   Start (position on chrom.): 127512178 End (position on chrom.): 127522054
> CELL
   PubMed on Product: carboxyl ester lipase-like (bile salt-stimulated)
   Start (position on chrom.): 127532733 End (position on chrom.): 127537549
> FS
   PubMed on Product: Forssman synthetase
   Start (position on chrom.): 127603661 End (position on chrom.): 127614093
> ABO blood group (transferase A, alpha)
   Start (position on chrom.): 127907180 End (position on chrom.): 127924298

### Haplotype 86-92

> BARHL1
> DDX31
> GTF3C4
> Q96MA6 (Adenylate cyclase)

### EXAMPLE 3: Detailed analysis of the region of the haplotype 86-92

Subsequent to the work presented in Example 2, the inventors continued the analysis of the region of chromosome 9 defined by the haplotype 86-92 still with the aid of the technology based on the SNPs in order to detect the alleles implicated in premature canities.

### 1 - Addition of 5 new SNPs in this region

In the region of the haplotype 86-92, such as defined at the conclusion of the individual genotyping carried out in Example 2, the following 5 SNPs are conserved:

| | | |
|---|---|---|
| SNP 86: 418620; | SNP 88: rs302919; | SNP 90: 932886; |
| SNP 91: 429269; | SNP 92: 2526008. | |

In a first stage, the inventors defined 5 new SNPs in this region in order to complete the preceding list. These 5 additional SNPs were selected from SNPs the polymorphism of which has already been validated by other research groups.

These 5 additional SNPs are numbered 86a, 86b, 86c, 86d and 91 a as a function of their relative position with respect to the 5 SNPs previously cited along chromosome 9 (from the telomer p towards the telomer q).

| | | |
|---|---|---|
| SNP 86a: 306537; | SNP 86b: 3739902; | SNP 86c: 371169; |
| SNP 86d: 3780813; | SNP 91a: rs106906. | |

In the case of the 10 SNPs thus defined, the "p-value" was calculated, i.e. the statistical difference between the groups Al and BI (Al: persons affected by PC with a phenotypic score of 4 or 5 and Bl: controls linked to the persons of group Al; see Example 2 for the exact definitions). Figure 8 illustrates the results obtained.

### 2 - Addition of 30 new SNPs in this region

At the conclusion of the previous step, in view of the particularly positive results concerning the linkage of the region 86-92 to the 'premature canities' phenotypic trait, the inventors decided to probe this region with a better supplied battery of SNPs.

They thus integrated 30 new SNPs in this region. Figure 11 reports the name of these 30 additional SNPs, their numbering from 1 to 30 as well as their relative position on chromosome 9 with respect to the 10 SNPs already defined. The table also indicates the re-numbering from 1 to 40 which was carried out for the total of the 30 additional SNPs and the 10 SNPs previously selected.

In the case of the 30 additional SNPs, the inventors also calculated the p value of the statistical deviation between the individuals of group Al and those of the group BI. Figure 9 illustrates the results obtained.

Finally, the inventors integrated the results on the p value obtained for the 40 SNPs covering the region 86 to 92. The results are illustrated in Figure 10. In this Figure, the axis of the abscissa which comprises the SNPs is graduated by taking into account the real intervals between the SNPs on chromosome 9.

Figure 11 also reports the p values (in fact -logp). It is recalled that a p value smaller than 0.001 indicates significant linkage.

The analysis of the results presented in Figure 11 shows that 4 SNPs present quite remarkable results with an association value -logp greater than 2.3. They are the SNPs: rs306534 (SNP 16/40); rs3739902 (SNP 25/40); rs575916 (SNP 30/40) and rs365297 (SNP 31/40). These SNPs, which present an association value with the premature canities trait are thus particularly indicated for any use linked to the diagnosis of premature canities.

### 3 - Analysis of the haplotypes

The inventors have observed that the SNPs of the region 86-92 are finally distributed in 2 regions, one region 86-88 and one region 90-92, which are in linkage disequilibrium.

The inventors hence then carried out a study of the association of these two haplotypes with the premature canities trait. The results are presented in the two tables of Figure 13.

It is apparent in this Figure that the results of association are very significant (p<10⁻⁵).

Starting from the results obtained on the haplotypes and from the excellent result obtained for the marker rs3739902 (-logp>3), the inventors analysed the region close to SNP rs3739902 more precisely in order to define a more precise haplotype showing a particularly close linkage to premature canities. The inventors were thus able to define the haplotype HAP25-27defined by the SNPs 25 to 27 (see Figure 10), the linkage score of which to premature canities is very high. The 3 SNPs 25 to 27 constituting the haplotype are rs3739902, rs2583805 and rs377090.

### EXAMPLE 4: Mutation scan in the region B 86-88

### Mutation scan in coding and non-coding sequences of the B86-88 region.

The strong association obtained by a cases-control study with trait PC, using a dense collection of SNP markers, encompasses a region of about 60 Kb of chromosome 9, as shown in the preceding examples. This region harbors two genes, *DDX31* and *GTF3C4.* In order to further investigate the potential functional role of this region in the PC trait, the inventors have performed a mutation scan in coding and splicing regions of both candidate genes (*DDX31,* 20 exons; *GTF3C4,* 5 exons).

They have sequenced also the entire 5'UTR region of DDX31 and GTF3C4 which stand in an area of 500 bp, between the first exon of these 2 genes, said area being supposed to contain promoters of both *DDX31* and *GTF3C4* genes.

In addition they have also determined the sequence of highly conserved regions (in comparison with mouse) that lie outside of coding areas (Conserved Non Genic regions, CNGs, see Dermitzakis et al. Science 2003) of both these genes in this 60 Kb of PC associated region that might have a functional role (regulation of gene expression, structure of DNA...).

### Methods

Each exon, intron-exon junction and non coding sequence DNA was individually amplified by PCR using primer pairs specific to each genome portion.

The data were determined by direct sequencing of DNA by the Sanger method. PCR products were purified individually before diDeoxy termination reaction. Sequenced fragments were resolved on an automatic 16 capillary DNA analyzer (Applied Biosystems, model 3100).

Sequencing data were analyzed using a DNA sequence alignment program, which allow to compare several sequences together.

Every nucleotide change from the reference sequence (sequence obtained by Human Genome Project) was recorded. Every non-silent variant, or potentially functionally important sequence change, was screened in a case and control population.

Population screening was performed either by direct sequencing or SNP genotyping (Pyrosequencing).

### DNA samples

The inventors have performed this analysis in DNA of individuals with PC and of controls. The affected individuals were from 6 families showing linkage of the PC trait with this region of chromosome 9. The additional 6 individuals were chosen among those having a high phenotypic score (see example 2, point 3 for the definition of phenotypic score of canities intensity). Six additional control individuals were also added to the analysis, for which the PC trait was formerly excluded.

### Results

A number of DNA variants were found in PC, controls or both groups of individuals. More variants were recorded in gene *DDX31* than *GTF3C4* (7 vs 2 variants), although both these genes have coding region of similar size (851 vs 822 residues).

| variant | location | | position | change |
|---|---|---|---|---|
| DDX31 | | | | |
| c.413G>A silent | exonic | 2 | c.413 | G>A |
| IVS3+15G>C | intronic | 3 | IVS3+15 | G>C |
| IVS4+15_17 delCTC | intronic | 4 | IVS4+15_17 | delCTC |
| IVS4+55C>T rs4498679 | intronic | 4 | IVS4+55 | C>T |
| IVS11-16_13 delCTTA | intronic | 11 | IVS11-16_13 | deICTTA |
| c.1674G>A rs306537 | exonic | 13 | c.1674 | |
| A800T | exonic | 20 | c.2398 | G>A |
| 1799V rs306547 | exonic | 20 | c.2395 | A>G |
| | | | | |

| GTF3C4 | | | | |
|---|---|---|---|---|
| c.36G>A, Silent | exonic | 1 | c.36 | G>A |
| c.1560A>G Silent | exonic | 3 | c.1560 | A>G |

The position of the nucleotides are given by reference to the start of the coding sequence, i.e. "c.413" means the 413^{th} nucleotide of the coding sequence, the 1^{st} nucleotide being the "A" of the codon ATG.

IVS stands for 'intervening sequence', i.e. exon. "IVS4+" identifies the intron 3' to the 4^{th} exon, whereas "IVS4-" identifies the intron 5' to the 4^{th} exon. "IVS4+15_17" identifies the 15^{th}, 16^{th} and 17^{th} nucleotides of the intron between exon 4 and 5, i.e. 3' to the 4^{th} exon. "A800T" and "I799V" are the mutations in the amino-acid sequence.

### Exonic variants

In gene DDX31, the inventors have identified 4 exonic variants (in exons 2, 13 and 20) and 3 intronic variants in a location close to the splicing site (lying in a distance range of 1-20 bp from splicing sites).

In gene GTF3C4, they have identified 2 exonic variants (in exons 2, 3) and no intronic variant close to the splicing site.

The only non-silent variants were found in exon 20 of gene DDX31. Variant on codon 799 was found as a translation change in protein DDX31 from amino-acid residue Isoleucine to Valine (I799V). This nucleotide variant is also known as a polymorphism (known as
SNP rs306547) that was found in 6 out of 12 affected individual DNAs in heterozygosity; 6 affected were showing homozygosity for this variant (V799). 1799V was identified in heterozygosity in all 6 controls. Overall there was no significant difference in the frequency of the respective genotypes and allelotypes between cases (64 individuals) and control (64 individuals) group of individual tested (table).

| **X20-I799V** | | | | | | | | | | |
|---|---|---|---|---|---|---|---|---|---|---|
| phenotypic score | GG | | GA | | AA | | G | | A | |
| | count | % | count | % | count | % | count % | | count | % |
| 5 | 12 | 60 | 7 | 35 | 1 | 5 | 31 | 77.5 | 9 | 22.5 |
| 4 | 25 | 56.8 | 17 | 38.6 | 2 | 4.5 | 67 | 65 | 36 | 35 |
| CON | 40 | 63.0 | 21 | 32.8 | 3 | 4.7 | 101 | 78.9 | 27 | 21.09 |

The other non-silent missense change in exon 20 of gene DDX31 (codon 800, Alanine changed to Threonine, A800T) was found in heteozygozity in one affected individual of the cohort. In order to estimate the potential effect of this variant A800T, a larger population of affected (64) and control (64) individuals was analysed and another carrier of this DNA variant was not found, in PC or controls. The DNA sequence codes for a substitution of a small amino acid by a small polar one. Residue at position 800 is not conserved through evolution since the homologous protein ddx31 residue in mouse is also a Threonine instead of Alanine in human.

| **X20-A800T** | | | | | | |
|---|---|---|---|---|---|---|
| phenotypic score | AA | | AG | | GG | |
| | count | % | count | % | count | % |
| 5 | 19 | 95 | 1 | 5 | 0 | 0 |
| 4 | 44 | 100.0 | 0 | 0.0 | 0 | 0.0 |
| | | | | | | |
| CON | 64 | 100.0 | 0 | 0.0 | 0 | 0.0 |

### Intronic variants

Another interesting variant was the deletion of trinucleotide CTC in a CTCCTC tandem repeated motif in intron 4 of gene DDX31 (IVS4+15_17deICTC). Interestingly, it was not possible to find this deleted CTC homozygously in any of the affected and control individuals tested.

The highest difference in frequency of heterozygous carrier for the del-allele was found in score-5 group of patient 23.8% compared to 9.26% in controls (162 reads) and 7.65% average genotype frequency in group of affected with a PC of score 1-4 and Piebaldism.

| **IVS4+15_17del** | | | | | | | |
|---|---|---|---|---|---|---|---|
| phenotypic score | AFF | AFF | AFF | CON | CON | CON | AFF vs CON |
| | + | total read | % | | total read | % | Fisher exact |
| 5 | 5 | 21 | 23.81 | | | | 0.04 |
| 4 | 4 | 48 | 8.33 | | | | |
| 3 | 2 | 49 | 4.08 | | | | |
| 2 | 1 | 18 | 5.56 | | | | |
| 1 | | | | | | | |
| p | 1 | 34 | 2.94 | | | | |
| all | 13 | 170 | 7.65 | 15 | 162 | 9.26 | |

### Putative promoter regions (in CpG island)

No variant was detected in the intergenic sequence located in both 5'UTR (genes GTF3C4 and DDX31 are oriented head to head from ATG codon). This region is identified as a CPG island.

### Conserved non genic regions

The inventors have also analyzed conserved non genic sequences (CNGs) that were identified in this locus. Out of the 20 CNGs the sequences of which were analyzed in the cohort of 12 affecteds+6 controls, only one variant was identified in the CNG called DDX31-CNGhs8, which lies in intron 18 of gene DDX31 (IVS18+3781-4397 / IVS19-1677-2293).

A comparison analysis of genotypic and allelic frequencies in 177 cases and 71 control DNAs showed that heterozygous genotype, i.e. genotype of combined alleles C and T is over represented in affected individual with phenotypic score 5 (45% vs 32%). This CNG is highly conserved from human to mouse, chicken and Fugu *(purple puffer).*

| DDX31-CNGhs8 affecteds+controls screening | | | | | | | | | | | | |
|---|---|---|---|---|---|---|---|---|---|---|---|---|
| plate # | CC | % CC | CT | % CT | TT | % TT | total genotypes | C | % C | T | % T | total allelotypes |
| AFF | | | | | | | | | | | | |
| GNXB01 GNXB02 | 46 | 0.51 | 39 | 0.43 | 5 | 0.06 | 90 | 131 | 0.73 | 49 | 0.27 | 180 |
| | 50 | 0.57 | 30 | 0.34 | 7 | 0.08 | 87 | 130 | 0.75 | 44 | 0.25 | 174 |
| CON | | | | | | | | | | | | |
| | | | | | | | | | | | | |
| GNXB03 | 44 | 0.62 | 23 | 0.32 | 4 | 0.06 | 71 | 111 | 0.78 | 31 | 0.22 | 142 |
| AFF scores | | | | | | | | | | | | |
| | | | | | | | | | | | | |
| 5+4 | 37 | 0.55 | 27 | 0.40 | 3 | 0.04 | 67 | 101 | 0.75 | 33 | 0.25 | 134 |
| 5 | 10 | 0.50 | 9 | 0.45 | 1 | 0.05 | 20 | 29 | 0.73 | 11 | 0.28 | 40 |

## Claims

1. Use of at least one SNP marker of the human chromosome 9, selected from the following markers: rs306534, rs3739902, rs575916 and rs365297, for the diagnosis of a predisposition to premature canities in an individual.

2. Use according to Claim 1, wherein the T allelic form of the SNP rs306534 indicates a predisposition to premature canities.

3. Use according to Claim 1, wherein the T allelic form of the SNP rs3739902 indicates a predisposition to premature canities.

4. Use according to Claim 1, wherein the G allelic form of the SNP rs575916 indicates a predisposition to premature canities.

5. Use according to Claim 1, wherein the T allelic form of the SNP rs365297 indicates a predisposition to premature canities.

6. Process for the diagnosis of a predisposition to premature canities in an individual, comprising the determination in a sample of the genetic material of said individual of the alleles of at least one SNP marker of the human chromosome 9, selected from the following markers: rs306534, rs3739902, rs575916 and rs365297.

7. Process according to Claim 6 comprising the additional step of comparison of the allelic form of the marker with that of other individuals in order to establish the diagnosis.

8. Process according to Claim 7 in which the other individuals are individuals who are not affected by premature canities.

9. Process according to Claim 7 wherein the other individuals are individuals who are affected by premature canities.

10. Process according to Claim 7 wherein the other individuals are individuals having a blood relationship with the individual to be diagnosed.

11. Process according to Claim 6, wherein the T allelic form of the SNP rs306534 indicates a predisposition to premature canities.

12. Process according to Claim 6, wherein the T allelic form of the SNP rs3739902 indicates a predisposition to premature canities.

13. Process according to Claim 6, wherein the G allelic form of the SNP rs575916 indicates a predisposition to premature canities.

14. Process according to Claim 6, wherein the T allelic form of the SNP rs365297 indicates a predisposition to premature canities.

15. Use of a means for detecting the alleles of a SNP marker of the human chromosome 9 selected from the following markers: rs306534, rs3739902, rs575916 and rs365297, to detect at least one allele of said SNP in a sample of the genetic material of an individual for the diagnosis of a predisposition to premature canities in that individual, wherein said means is a nucleic acid probe.

16. Use according to Claim 15 wherein said probe is coupled to a radioactive, enzymatic, luminescent or fluorescent marker.

17. Use according to Claim 15, wherein said means makes it possible to determine the T allelic form of the SNP rs306534.

18. Use according to Claim 15, wherein said means makes it possible to determine the T allelic form of the SNP rs3739902.

19. Use according to Claim 15, wherein said means makes it possible to determine the G allelic form of the SNP rs575916.

20. Use according to Claim 15, wherein said means makes it possible to determine the T allelic form of the SNP rs365297.

21. Use according to Claim 15, wherein several means are used to detect the alleles of at least two distinct SNPs selected from rs306534, rs3739902, rs575916 and rs365297.

22. Kit for the diagnosis of a predisposition to premature canities, containing:
- several means for detecting in a sample of human genetic material, the alleles of at least two SNP markers of the human chromosome 9 selected from the markers rs306534, rs3739902, rs575916 and rs365297 and
- a positive or negative control,
wherein said means are hybridisation probes hybridising specifically with one allele of the chosen SNP.

23. Kit according to Claim 22, wherein said probes are coupled to a radioactive, enzymatic, luminescent or fluorescent marker.

24. Kit according to claim 22, containing means for detection the alleles of at least three SNP markers of the human chromosome 9 selected from the markers rs306534, rs3739902, rs575916 and rs365297.

25. Kit according to Claim 22, wherein at least one means makes it possible to determine the T allelic form of the SNP rs306534.

26. Kit according to Claim 22, wherein at least one means makes it possible to determine the T allelic form of the SNP rs3739902.

27. Kit according to Claim 22, wherein at least one means makes it possible to determine the G allelic form of the SNP rs575916.

28. Kit according to Claim 22, wherein at least one means makes it possible to determine the T allelic form of the SNP rs365297.

29. Process for the diagnosis of a predisposition to premature canities in an individual, comprising the determination, in a sample of the genetic material of said individual, of the alleles of the following 3 SNPs markers of the human chromosome 9: rs3739902, rs2583805 and rs377090, in order to determine the haplotype of that individual relative to these 3 SNPs.

30. Process according to Claim 29 including the additional step of comparing the haplotype formed by the 3 SNPs to that of other individuals in order to establish the diagnosis.

31. Process according to Claim 30 wherein the other individuals are individuals who are not affected by premature canities.

32. Process according to Claim 30 wherein the other individuals are individuals who are affected by premature canities.

33. Process according to Claim 30 wherein the other individuals are individuals who have a blood relationship to the individual to be diagnosed.

34. Use of means for detecting the alleles of three SNPs markers of the human chromosome 9 said markers being rs3739902, rs2583805 and rs377090 to detect the alleles of each of the three SNPs in a sample of the genetic material of an individual for the diagnosis of a predisposition to premature canities in that individual, wherein said means are nucleic acid probes.

35. Use according to Claim 34, wherein said probes are coupled to radioactive, enzymatic, luminescent or fluorescent markers.

36. Kit for the diagnosis of a predisposition to premature canities, containing means for detecting in a sample of human genetic material, the alleles of each of the following SNP markers of the human chromosome 9: rs3739902, rs2583805 and rs377090,
wherein means are hybridisation probes hybridising specifically with one given allele of each SNP marker.

37. Kit according to Claim 36, containing in addition a positive or negative control.

38. Kit according to Claim 36, wherein said probes are coupled to radioactive, enzymatic, luminescent or fluorescent markers.

## Patentansprüche

1. Verwendung mindestens eines SNP-Markers des menschlichen Chromosom 9, ausgewählt aus den folgenden Markern: rs306534, rs3739902, rs575916 und rs365297, zur Diagnose einer Prädisposition vorzeitiger Canities in einem Individuum.

2. Verwendung gemäß Anspruch 1, worin die T-allelische Form des SNP rs306534 die Prädisposition vorzeitiger Canities anzeigt.

3. Verwendung gemäß Anspruch 1, worin die T-allelische Form des SNP rs3739902 die Prädisposition vorzeitiger Canities anzeigt.

4. Verwendung gemäß Anspruch 1, worin die G-allelische Form des SNP rs575916 die Prädisposition vorzeitiger Canities anzeigt.

5. Verwendung gemäß Anspruch 1, worin die T-allelische Form des SNP rs365297 die Prädisposition vorzeitiger Canities anzeigt.

6. Verfahren zur Diagnose einer Prädisposition vorzeitiger Canities in einem Individuum, wobei man in einer Probe des genetischen Materials des genannten Individuum die Allele mindestens eines SNP-Markers des menschlichen Chromosom 9, ausgewählt aus den folgenden Markern: rs306534, rs3739902, rs575916 und rs365297, bestimmt.

7. Verfahren gemäß Anspruch 6, welches die zusätzliche Stufe zum Vergleich der allelischen Form des Markers mit derjenigen weiterer Individuen zur Erstellung der Diagnose umfasst.

8. Verfahren gemäß Anspruch 7, in welchem die weiteren Individuen von der vorzeitigen Canities nicht betroffene Individuen sind.

9. Verfahren gemäß Anspruch 7, worin die weiteren Individuen von der vorzeitigen Canities betroffene Individuen sind.

10. Verfahren gemäß Anspruch 7, worin die weiteren Individuen Individuen mit einer Blutsbeziehung zu dem Individuum sind, der diagnostiziert wird.

11. Verfahren gemäß Anspruch 6, worin die T-allelische Form des SNP rs306534 die Prädisposition vorzeitiger Canities anzeigt.

12. Verfahren gemäß Anspruch 6, worin die T-allelische Form des SNP rs3739902 die Prädisposition vorzeitiger Canities anzeigt.

13. Verfahren gemäß Anspruch 6, worin die G-allelische Form des SNP rs575916 die Prädisposition vorzeitiger Canities anzeigt.

14. Verfahren gemäß Anspruch 6, worin die T-allelische Form des SNP rs365297 die Prädisposition vorzeitiger Canities anzeigt.

15. Verwendung eines Mittels zum Nachweis der Allele eines SNP-Markers des menschlichen Chromosom 9, ausgewählt aus den folgenden Markern: rs306534, rs3739902, rs575916 und rs365297, zum Nachweis mindestens eines Allel des genannten SNP in einer Probe des genetischen Materials eines Individuum zur Diagnose einer Prädisposition vorzeitiger Canities in diesem Individuum, worin das genannte Mittel eine Nucleinsäure-Sonde ist.

16. Verwendung gemäß Anspruch 15, worin die genannte Sonde an einen radioaktiven, enzymatischen, lumineszenten oder fluoreszenten Marker gekuppelt ist.

17. Verwendung gemäß Anspruch 15, worin das genannte Mittel die Bestimmung der T-allelischen Form des SNP rs306534 ermöglicht.

18. Verwendung gemäß Anspruch 15, worin das genannte Mittel die Bestimmung der T-allelischen Form des SNP rs3739902 ermöglicht.

19. Verwendung gemäß Anspruch 15, worin das genannte Mittel die Bestimmung der G-allelischen Form des SNP rs575916 ermöglicht.

20. Verwendung gemäß Anspruch 15, worin das genannte Mittel die Bestimmung der T-allelischen Form des SNP rs365297 ermöglicht.

21. Verwendung gemäß Anspruch 15, worin mehrere Mittel zum Nachweis der Allele von mindestens zwei unterschiedlichen SNPs, ausgewählt aus rs306534, rs3739902, rs575916 und rs365297, verwendet werden.

22. Kit zur Diagnose einer Präposition vorzeitiger Canities, enthaltend:
- mehrere Mittel zum in einer Probe menschlichen genetischen Materials durchgeführten Nachweis der Allele von mindestens zwei SNP-Markern des menschlichen Chromosom 9, ausgewählt aus den Markern rs306534, rs3739902, rs575916 und rs365297, und
- einen Positiv- oder Negativ-Vergleich,
worin die genannten Mittel Hybridisierungssonden sind, die spezifisch mit einem Allel des gewählten SNP hybridisieren.

23. Kit gemäß Anspruch 22, worin die genannten Sonden an einen radioaktiven, enzymatischen, lumineszenten oder fluoreszenten Marker gekuppelt sind.

24. Kit gemäß Anspruch 22, enthaltend Mittel zum Nachweis der Allele von mindestens drei SNP-Markern des menschlichen Chromosom 9, ausgewählt aus den Markern rs306534, rs3739902, rs575916 und rs365297.

25. Kit gemäß Anspruch 22, worin mindestens ein Mittel die Bestimmung der T-allelischen Form des SNP rs306534 ermöglicht.

26. Kit gemäß Anspruch 22, worin mindestens ein Mittel die Bestimmung der T-allelischen Form des SNP rs3739902 ermöglicht.

27. Kit gemäß Anspruch 22, worin mindestens ein Mittel die Bestimmung der G-allelischen Form des SNP rs575916 ermöglicht.

28. Kit gemäß Anspruch 22, worin mindestens ein Mittel die Bestimmung der T-allelischen Form des SNP rs365297 ermöglicht.

29. Verfahren zur Diagnose einer Prädisposition vorzeitiger Canities in einem Individuum, wobei man in einer Probe des genetischen Materials des genannten Individuum die Allele der folgenden drei SNP-Marker des menschlichen Chromosom 9: rs3739902, rs2583805 und rs377090 zur Bestimmung des Haplotyps dieses Individuum bezüglich dieser 3 SNPs bestimmt.

30. Verfahren gemäß Anspruch 29, enthaltend die zusätzliche Stufe zum Vergleich des mit den 3 SNPs gebildeten Haplotyps mit demjenigen weiterer Individuen zur Erstellung der Diagnose.

31. Verfahren gemäß Anspruch 30, worin die weiteren Individuen von der vorzeitigen Canities nicht betroffene Individuen sind.

32. Verfahren gemäß Anspruch 30, worin die weiteren Individuen von der vorzeitigen Canities betroffene Individuen sind.

33. Verfahren gemäß Anspruch 30, worin die weiteren Individuen Individuen mit einer Blutbeziehung zu dem Individuum sind, der diagnostiziert wird.

34. Verwendung von Mitteln zum Nachweis der Allele von 3 SNP-Markern des menschlichen Chromosom 9, wobei die genannten Marker rs3739902, rs2583805 und rs377090 sind, zum Nachweis der Allele eines jeden der 3 SNPs in einer Probe des genetischen Materials eines Individuum für die Diagnose einer Prädisposition vorzeitiger Canities in diesem Individuum, worin die genannten Mittel Nucleinsäure-Sonden sind.

35. Verwendung gemäß Anspruch 34, worin die genannten Sonden an radioaktive enzymatische, lumineszente oder fluoreszente Marker gekuppelt sind.

36. Kit zur Diagnose einer Prädisposition vorzeitiger Canities, enthaltend Mittel zum in einer Probe des menschlichen genetischen Materials durchgeführten Nachweis der Allele eines jeden der folgenden SNP-Marker des menschlichen Chromosom 9: rs3739902, rs2583805 und rs377090, worin die Mittel Hybridisierungssonden sind, die spezifisch mit einem gegebenen Allel des jeweiligen SNP-Markers hybridisieren.

37. Kit gemäß Anspruch 36, zusätzlich enthaltend einen Positiv- oder Negativ-Vergleich.

38. Kit gemäß Anspruch 36, worin die genannten Sonden an radioaktive, enzymatische, lumineszente oder fluoreszente Marker gekuppelt sind.

## Revendications

1. Utilisation d'au moins un marqueur SNP du chromosome 9 humain, choisi parmi les marqueurs suivants : rs306534, rs3739902, rs575916 et rs365297, pour le diagnostic d'une prédisposition à la canitie précoce chez un individu.

2. Utilisation selon la revendication 1, où la forme allélique T du SNP rs306534 indique une prédisposition à la canitie précoce.

3. Utilisation selon la revendication 1, où la forme allélique T du SNP rs3739902 indique une prédisposition à la canitie précoce.

4. Utilisation selon la revendication 1, où la forme allélique G du SNP rs575916 indique une prédisposition à la canitie précoce.

5. Utilisation selon la revendication 1, où la forme allélique T du SNP rs365297 indique une prédisposition à la canitie précoce.

6. Procédé pour le diagnostic d'une prédisposition à la canitie précoce chez un individu, comprenant la détermination, dans un échantillon du matériel génétique dudit individu, des allèles d'au moins un marqueur SNP du chromosome 9 humain, choisi parmi les marqueurs suivants : rs306534, rs3739902, rs575916 et rs365297.

7. Procédé selon la revendication 6 comprenant l'étape supplémentaire de comparaison de la forme allélique du marqueur à celle d'autres individus pour établir le diagnostic.

8. Procédé selon la revendication 7 où les autres individus sont des individus qui ne sont pas atteints de canitie précoce.

9. Procédé selon la revendication 7 où les autres individus sont des individus qui sont atteints de canitie précoce.

10. Procédé selon la revendication 7 où les autres individus sont des individus ayant un lien sanguin avec l'individu à diagnostiquer.

11. Procédé selon la revendication 6, où la forme allélique T du SNP rs306534 indique une prédisposition à la canitie précoce.

12. Procédé selon la revendication 6, où la forme allélique T du SNP rs3739902 indique une prédisposition à la canitie précoce.

13. Procédé selon la revendication 6, où la forme allélique G du SNP rs575916 indique une prédisposition à la canitie précoce.

14. Procédé selon la revendication 6, où la forme allélique T du SNP rs365297 indique une prédisposition à la canitie précoce.

15. Utilisation d'un moyen pour détecter les allèles d'un marqueur SNP du chromosome 9 humain choisi parmi les marqueurs suivants : rs306534, rs3739902, rs575916 et rs365297, en vue de détecter au moins un des allèles dudit SNP dans un échantillon du matériel génétique d'un individu, pour le diagnostic d'une prédisposition à la canitie précoce chez cet individu, où ledit moyen est une sonde nucléique.

16. Utilisation selon la revendication 15 où ladite sonde est couplée à un marqueur radioactif, enzymatique, luminescent ou fluorescent.

17. Utilisation selon la revendication 15, où ledit moyen permet de déterminer la forme allélique T du SNP rs306534.

18. Utilisation selon la revendication 15, où ledit moyen permet de déterminer la forme allélique T du SNP rs3739902.

19. Utilisation selon la revendication 15, où ledit moyen permet de déterminer la forme allélique G du SNP rs575916.

20. Utilisation selon la revendication 15, où ledit moyen permet de déterminer la forme allélique T du SNP rs365297.

21. Utilisation selon la revendication 15, où plusieurs moyens sont utilisés pour détecter les allèles d'au moins deux marqueurs SNPs distincts choisis parmi rs306534, rs3739902, rs575916 et rs365297.

22. Kit pour le diagnostic d'une prédisposition à la canitie précoce, comprenant
- plusieurs moyens pour détecter, dans un échantillon de matériel génétique humain, les allèles d'au moins deux marqueurs SNPs du chromosome 9 humain choisis parmi les marqueurs rs306534, rs3739902, rs575916 et rs365297 et
- un contrôle positif ou négatif,
où lesdits moyens sont des sondes d'hybridation s'hybridant spécifiquement avec un allèle d'un marqueur SNP choisi.

23. Kit selon la revendication 22, où lesdites sondes sont couplées à un marqueur radioactif, enzymatique, luminescent ou fluorescent.

24. Kit selon la revendication 22, contenant des moyens pour la détection des allèles d'au moins 3 marqueurs SNPs du chromosome 9 humain choisis parmi rs306534, rs3739902, rs575916 et rs365297.

25. Kit selon la revendication 22, où au moins un desdits moyens permet de déterminer la forme allélique T du SNP rs306534.

26. Kit selon la revendication 22, où au moins un desdits moyens permet de déterminer la forme allélique T du SNP rs3739902.

27. Kit selon la revendication 22, où au moins un desdits moyens permet de déterminer la forme allélique G du SNP rs575916.

28. Kit selon la revendication 22, où au moins un desdits moyens permet de déterminer la forme allélique T du SNP rs365297.

29. Procédé pour le diagnostic d'une prédisposition à la canitie précoce chez un individu, comprenant la détermination, dans un échantillon du matériel génétique dudit individu, des allèles des 3 marqueurs SNPs du chromosome 9 humain suivants : rs3739902, rs2583805 et rs377090, afin de déterminer l'haplotype de cet individu relatif à ces 3 SNPs.

30. Procédé selon la revendication 29 comprenant l'étape supplémentaire de comparaison de l'haplotype formé par les 3 SNPs à celui d'autres individus pour établir le diagnostic.

31. Procédé selon la revendication 30 où les autres individus sont des individus qui ne sont pas atteints de canitie précoce.

32. Procédé selon la revendication 30 où les autres individus sont des individus qui sont atteints de canitie précoce.

33. Procédé selon la revendication 30 où les autres individus sont des individus qui ont un lien sanguin avec l'individu à diagnostiquer.

34. Utilisation de moyens pour détecter les allèles des trois marqueurs SNPs du chromosome 9 humain, lesdits marqueurs étant rs3739902, rs2583805 et rs377090, en vue de détecter les allèles de chacun des 3 marqueurs SNPs dans un échantillon du matériel génétique d'un individu, pour le diagnostic d'une prédisposition à la canitie précoce chez cet individu, où lesdits moyens sont des sondes nucléiques.

35. Utilisation selon la revendication 34 où lesdites sondes sont couplées à des marqueurs radioactifs, enzymatiques, luminescents ou fluorescents.

36. Kit pour le diagnostic d'une prédisposition à la canitie précoce, comprenant des moyens pour détecter, dans un échantillon de matériel génétique humain, les allèles de chacun des marqueurs SNPs du chromosome 9 humain suivants : rs3739902, rs2583805 et rs377090, où lesdits moyens sont des sondes d'hybridation s'hybridant spécifiquement avec un allèle donné de chaque marqueur SNP.

37. Kit selon la revendication 36, comprenant de plus un contrôle positif ou négatif.

38. Kit selon la revendication 36, où lesdites sondes sont couplées à des marqueurs radioactifs, enzymatiques, luminescents ou fluorescents.
